# EUROPEAN PATENT SPECIFICATION

(11) **EP 3 395 956 B1**
(45) Date of publication and mention of the grant of the patent: **13.11.2019**
(21) Application number: 18169749.1
(22) Date of filing: 27.04.2018
(51) Int. Cl.: C12Q 1/68

(54) **METHOD FOR DETERMINING THE AMOUNT OF DNA IN SAMPLE**
VERFAHREN ZUR BESTIMMUNG DER DNS MENGE IN EINER PROBE
PROCÉDÉ POUR LA DÉTERMINATION DE LA QUANTITÉ D'ADN DANS UN ÉCHANTILLON

(30) Priority: 27.04.2017 CZ 20170233
(43) Date of publication of application: 31.10.2018
(73) Proprietor: Univerzita Palackého v Olomouci, 771 47 Olomouc (CZ)
(72) Inventor: Ligasová, Anna, 77900 Olomouc (CZ); Koberna, Karel, 77900 Olomouc (CZ)
(74) Representative: Hartvichova, Katerina

(56) References cited:
- US-A1- 2013 045 485
- DECLERCK P ET AL: "A Detection Method For Legionella Spp In (Cooling) Water: Fluorescent In Situ Hybridisation (FISH) On Whole Bacteria", WATER SCIENCE AND TECHNO,, vol. 47, no. 3, 1 January 2003 (2003-01-01), pages 143-146, XP008077404, ISSN: 0273-1223
- MANINDRA NATH TIWARI ET AL: "Nicotine-encapsulated poly(lactic-co-glycolic) acid nanoparticles improve neuroprotective efficacy against MPTP-induced parkinsonism", FREE RADICAL BIOLOGY AND MEDICINE, vol. 65, 1 December 2013 (2013-12-01), pages 704-718, XP055231325, US ISSN: 0891-5849, DOI: 10.1016/j.freeradbiomed.2013.07.042
- DATABASE MEDLINE [Online] US NATIONAL LIBRARY OF MEDICINE (NLM), BETHESDA, MD, US; March 2016 (2016-03), XIAO SHU-WEI ET AL: "[A perfusion system for preparing canine ureteral acellular matrix].", XP002783707, Database accession no. NLM27063164 & NAN FANG YI KE DA XUE XUE BAO = JOURNAL OF SOUTHERN MEDICAL UNIVERSITY MAR 2016, vol. 36, no. 3, March 2016 (2016-03), pages 365-370, ISSN: 1673-4254
- Guoping Chen ET AL: "Decellularization Techniques for Tissue Engineering", , 12 March 2015 (2015-03-12), pages 1-13, XP055274973, DOI: 10.1002/9780470027318.a9472 Retrieved from the Internet: URL:http://onlinelibrary.wiley.com/store/1 0.1002/9780470027318.a9472/asset/a9472.pdf ?v=1&t=iolfv7s7&s=5caf9b015580a8cd9520c7bd e421a49605183c84 [retrieved on 2016-05-24]
- DATABASE BIOSIS, [Online] 16 October 2007 (2007-10-16), MATTHIESEN THOMAS S ET AL: "Large solid organ perfusion decellularization - A start for human-sized tissue scaffolds?", XP002612655, retrieved from BIOSIS Database accession no. PREV200800197464

## Description

### Field of Art

The present invention concerns the determination of the amount of DNA in samples and use thereof for determining the number of cells.

### Background Art

A number of methods are used for determining the amount of DNA in samples, see for example US 2013/045485 disclosing a method of determining the amount of DNA in a sample using a DNA-binding dye. The most frequent method is the isolation of DNA from a sample and the subsequent measurement of the concentration using the absorbance of certain wavelengths. This technology, however, requires the isolation of the DNA and is therefore not very suitable e.g. for determining the number of cells or in cases when it is DNA otherwise bound in the sample, e.g. on the surface of the well plates. In the case of the determination of the number of cells, one of the most widespread methods is the variant, which relies on the direct counting of the cells using a microscope and haemocytometer. Although it is an inexpensive method, its main disadvantage is its time-demanding nature. Alternative methods for determining the number of cells were therefore developed and are still being developed. These approaches are based e.g. on the conversion of some substrates using cellular enzymes and measurement of the changes of the concentration of the product of this reaction. The examples are the methods labelled as MTT, MTS, WST-1 and the methods based on alamar blue assays (J. Cell. Mol. Med., 14, 1003-1013). The disadvantage of the mentioned methods is their dependence on the metabolic state of the cell population. Among others, it could be related also to the density of the cell population (Tissue Eng., 11, 182-191) and thus does not have to provide the linear course of the dependence of the signal on the number of cells. Therefore, methods based precisely on the detection of cellular DNA were developed and are being used. An example are the fluorescent substances, which bind to DNA and after their binding to DNA there is a significant increase of their fluorescence. A typical example are the cyanine dyes CyQuant and PicoGreen (J. Immunol. Methods, 254, 85-98). Methods based on DAPI (4',6- diamidino-2-phenylindole; Biotech. Histochem., 70, 220-233, Scheme 1) or Hoechst stains (In Vitro Cell Dev. Biol., 24, 247-252; J. Immunol. Methods, 162, 41-45; J. Histochem. Cytochem., 23, 493-505; J. Histochem. Cytochem., 24, 24-33; Scheme 1) were elaborated on a similar principle. The minimal number of cells, which can be determined by the individual methods differs and is generally in the range from several tens to 1,000 cells in a well of a 96-well plate. The disadvantage of these methods is the need to assure measurement in the entire well or in the total volume of the sample to ensure the highest possible precision of the measurement. The individual methods also differ in the speed of their performance, the possibilities of observing other parameters, e.g. replication activity and material demands, where the decisive factor is not in a number of applications the sensitivity of the methods but rather the need to assure a high linearity in a broad framework of cellular concentrations.

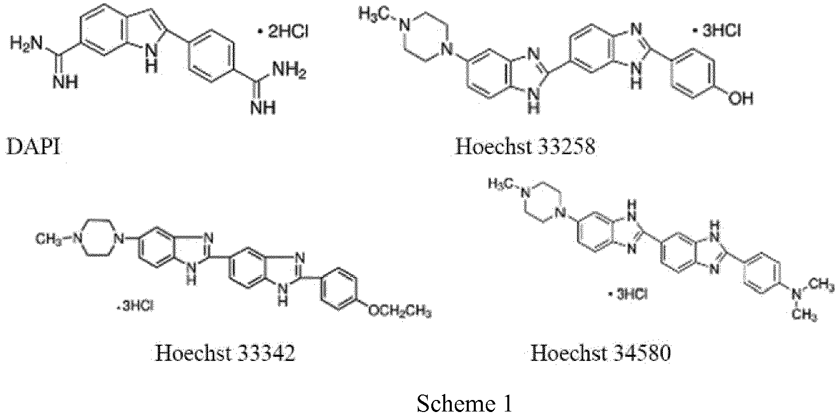

### Disclosure of the Invention

The present invention describes a method of determining the amount of DNA in samples, in which this DNA is anchored. An example of samples are predominantly samples containing DNA, which is anchored on particles or surfaces, and samples containing cells. An example of particles can be magnetic or silicon or glass particles, an example of the surfaces could be the surfaces of microtitration plates. Samples containing cells could be e.g. tissues, cells attached to supports of the type of Petri dishes, cultivation flasks, microscopic glasses or multiple-well plates or cells in a suspension culture. Since the amount of DNA in cells is directly proportional to their number, the method is applicable for determining the number of cells in samples containing cells. As in the case of samples containing anchored DNA, also in the case of samples containing cells, the described method provides a linear dependence of the signal on the amount of DNA in a wide range of values. For instance, it provides completely linear results in 96-well plates already from ca 100 cells through a fully-grown well.

The described method is based on labelling the DNA using fluorescent substances, which bind to DNA, washing the samples in the washing solution and washing out of the fluorescent substances using an elution solution. The fluorescence measurement then takes place in the elution solution containing the fluorescent substances eluted from the DNA, and which may, but not necessarily does, contain also the DNA being determined. The incubation of samples with fluorescent substances, their washing in the washing solution and elution of the fluorescent substances using the elution solution are the basic steps of the described method. These steps may, but not necessarily do, follow closely one after another. Thanks to the suitably selected composition of the elution solutions, which ensure high washing out, linearity of the dependence of the signal on the amount of DNA and at the same the high fluorescence of the fluorescent substances used, the fluorescent signal measured in the elution solution reflects the amount of DNA.

The object of the present invention is a method of determining the amount of DNA in a sample, which contains the following steps:
(i) a sample is incubated in a solution of at least one fluorescent substance selected from the group consisted of 4',6-diamidino-2-phenylindole (DAPI, Scheme 1; Biotech. Histochem., 70, 220-233), 2-(4-hydroxyphenyl)-5-(4-methyl-1-piperazinyl)-2,5-bi-1H-benzimidazole (Hoechst 33258), 2-(4-ethoxyphenyl)-5-(4-methyl-1-piperazinyl)-2,5-bi-1H-benzimidazole (Hoechst 33342) and N,N-dimethyl-4-[5-(4-methyl-1-piperazinyl)[2,5'-bi-1H-benzimidazole]-2'-yl]benzenamine (Hoechst 34580, Scheme 1; J. Histochem. Cytochem., 23, 493-505, J. Histochem. Cytochem., 24, 24-33, Cytometry, 44, 133-136), which, in the course of this incubation, binds to the DNA present in the sample;
(ii) the sample is washed with an aqueous washing solution and/or water, by which the fluorescent substances not bound to the DNA are washed away from the sample;
(iii) the fluorescent substances bound to the DNA in the sample are eluted from the DNA with an aqueous elution solution of at least one substance selected from the group consisted of sodium dodecylsulphate, lithium dodecylsulphate and sodium [dodecanoyl(methyl)amino]acetate, wherein the concentration of sodium dodecylsulphate, lithium dodecylsulphate and/or sodium [dodecanoyl(methyl)amino]acetate is at least 0.1 % (w/v);
(iv) the amount of DNA is determined in the elution solution from the previous step by measuring the fluorescent signal of the fluorescent substance used in step (i).

In the described method, the samples containing DNA are incubated in a solution, preferably an aqueous solution, of these fluorescent substances: DAPI and/or Hoechst 33258 and/or Hoechst 33342 and/or Hoechst 34580 (hereinafter only Hoechst, which denotes Hoechst 33258, Hoechst 33342, Hoechst 34580 or combinations thereof) These substances are commonly used for the detection and quantification of DNA thanks to the significant increase of fluorescence after their binding to the molecules of DNA. Although from the perspective of price and manipulation, it is preferable to use the aqueous solution of DAPI and/or Hoechst, it is possible to use even other solutions. The examples are DAPI and/or Hoechst solutions in ethanol, methanol or dimethylsulfoxide (DMSO). It is also possible to use mixtures of various solutions and solvents. An example is a mixture of ethanol and water or ethanol and aqueous solutions or a mixture of methanol and aqueous solutions. In the case of cell cultures, the incubation with DAPI and/or Hoechst can be conducted e.g. in a growth medium of cells or after their further processing. In the case of pieces of tissues, incubation with DAPI and/or Hoechst can also be conducted immediately after removal from the organism or after further processing An example of further processing is the drying, fixation e.g. in formaldehyde or ethanol, permeabilization with detergents, or incubation in a solution of monovalent copper. These steps can be mutually combined. Generally, the method can be used for variously processed samples containing DNA.

In one preferred embodiment of the present invention, the optimal concentration of DAPI and/or Hoechst for determination the amount of DNA is from 0.1 to 20 µmol.l⁻¹.

One part of the described method is the washing step, which is performed after the incubation of the samples with DAPI and/or Hoechst. In its course, the non-specifically bound fluorescent substances used - DAPI and/or Hoechst - are removed. They could be non-specifically bound e.g. to the samples, the surfaces of incubation vessels and their components, possibly other parts, which were incubated with DAPI and/or Hoechst in the course of the application of the method. They could be, for instance, coverslips or microscopic slides or filters. According to our findings, for instance, the surfaces of common polystyrene cultivation vessels bind relatively strongly DAPI and also Hoechst. The incubation of samples for a period of 5 minutes to one hour, preferably for at least 10 minutes, is usually sufficient, with three to four changes of the washing solution. In the case that these fluorescent substances would not be perfectly removed, it would cause an increase of a non-specific signal, which would lead to a decrease of the sensitivity of the method.
Although it was possible to use water for the washing step, washing solutions based on aqueous solutions of buffers with a pH preferably between 6 to 8, which preferably at the same time contained at least one detergent, selected from the group comprising polyoxyethylenesorbitan monolaurate (Tween 20, Scheme 2), polyoxyethylenesorbitan monooleate (Tween 80, Scheme 2), 4-(1,1,3,3-tetramethylbutyl)phenyl-polyethylene glycol (Triton X 100, Scheme 2), saponins (a group of amphipathic glycosides), 4-nonylphenyl-polyethylen glycol (Nonidet P 40, Scheme 2), sodium dodecylsulphate (NaC₁₂H₂₅SO₄, SDS), lithium dodecylsulphate (LiC₁₂H₂₅SO₄, LiDS), and sodium [dodecanoyl(methyl)amino]acetate (C₁₅H₂₈NNaO₃, sarkosyl), have shown to be preferential.

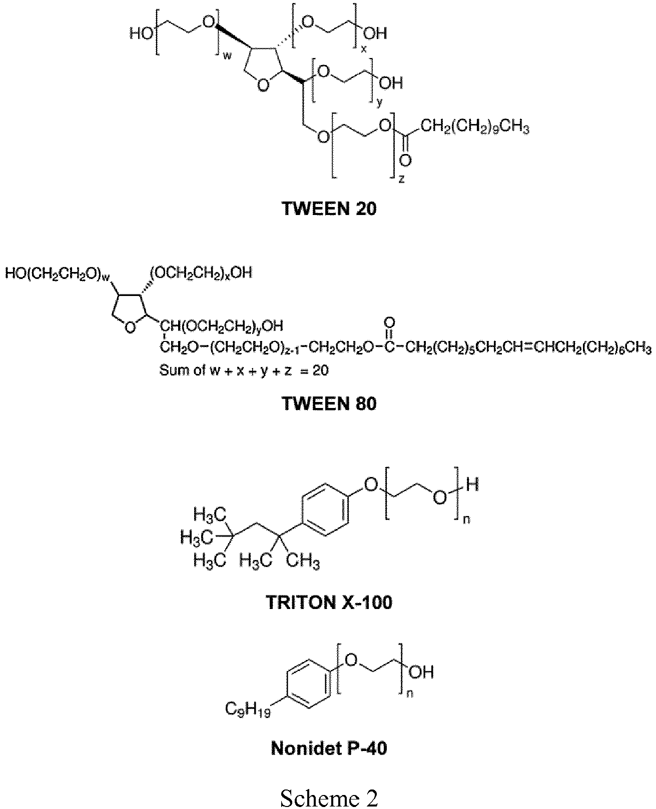

A sufficient concentration of the detergent for washing the sample is in the range of from 0.01 % (vol./vol.) to 10 % (vol./vol.), preferably in the range of from 0.05 % (vol./vol.) to 5 % (vol./vol.). In the case, that the detergent is sodium dodecylsulphate, lithium dodecylsulphate, and/or sodium [dodecanoyl(methyl)amino] acetate, the concentration thereof in the washing solution is in the range of from 0.01 to 0.05 % (w/v). Of the buffers, for instance a phosphate buffer prepared from monosodium dihydrogen phosphate and dipotassium hydrogen phosphate or monosodium dihydrogen-phosphate and dipotassium hydrogen phosphate or potassium dihydrogen phosphate and disodium hydrogen phosphate, or a phosphate buffer containing sodium chloride and/or potassium chloride and/or magnesium chloride is preferable in terms of price. Other buffers were also tested successfully, e.g. buffers containing Tris (2-amino-2-(hydroxymethyl)propane-1,3-diol) or HEPES (2-[4-(2-hydroxyetyl)piperazine-1-yl]ethanesulfonic acid). We generally did not observe that any of the tested buffers would show a significant influence on the speed and efficiency of the washing. On the contrary, the additive of detergents increased the efficiency. In order to maximally eliminate the non-specific signal derived from the non-specifically bound DAPI and/or Hoechst molecules, the whole procedure is preferably performed also in control samples without DNA and the signal measured in these samples is then subtracted from the signal of the sample containing DNA.

Because both DAPI and Hoechst may, to the various degree of extent, bind also to RNA, it is preferable in the cases, when the exact determination of DNA is necessary and the samples contain also RNA, to eliminate the RNA, for example using RNase, preferably RNase A. In one preferred embodiment of the present invention, before the step (i) of incubation of the sample with DAPI and/or Hoechst and/or before the step (ii) of washing the sample with the aqueous washing solution and/or water, and/or before the step (iii) of elution of the sample using elution solution, incubation of the sample with RNase, preferably with RNase A, is performed. The used concentration of RNase A is preferably in the range from 0.1 to 0.5 mg.ml⁻¹. The samples are incubated with RNase A preferably for 10 to 60 minutes at the temperature from 20 to 37 °C. Optimally, this step is performed before the washing step.

In the step (iii), the fluorescence substances bound to DNA are eluted from DNA using aqueous elution solution, preferably buffered solution, of at least one of the compounds selected from the group consisting of SDS and/or LiDS and/or sarkosyl; Systematical names of these compounds are mentioned above, and can be called elution substances in the following text. This step assures the washing out of the fluorescent substances bound to the DNA into the solution. The incubation leads to the full washing out of the fluorescent substances both from the DNA bound to the carrier and from the cells. The concentration of the elution substances is decisive for a thorough wash out. The minimal concentrations, which lead to the effective wash out of DAPI and Hoechst are 0.1 % (weight/vol.). Preferably, these concentrations are from 0.1 % to 10 % (weight/vol.) of SDS and LiDS and from 1 % to 10 % (weight/vol.) of sarkosyl. For the majority of the tested protocols, 0.5 % - 2 % (weight/vol.) of the solution of the elution substances was sufficient for the effective elution of the fluorescent substances.

It is generally true that with the increasing concentrations of the elution substances, there was an increase of the speed of elution. It was possible to use also a combination of more than one elution substances. Elution of the largest part of the fluorescent substances took place most often in the course of a few minutes. Only in the case of dried specimen it was necessary to use times, which ran from several tens of minutes to an hour.

It is also possible to use the elution substances in low concentrations, when there has not been an effective elution of the fluorescent substances from the DNA, in a washing solution of the washing step (ii), which precedes the elution step (iii). In this case, washing solutions contain a maximum of 0.05 % (weight/vol.) of the elution substances and, at the same time, the concentrations of the elution substances used in the washing solution are preferably at least 10x lower than their concentrations used in the elution solution. Such low concentrations do not have a significant impact on the error of determining the amount of DNA, because only a negligible amount of the fluorescent substances is eluted during the washing step.

In one preferred embodiment of the present invention, the sample is cells or tissues or anchored DNA, wherein in the case that the sample is cells or tissues, the sample is preferably fixed. It is possible to use a wide range of approaches for the fixation of the cells. The cells could be, for instance, fixed by a solution of formaldehyde, preferably with aqueous 1 to 8 % (weight/vol.) buffered solution of formaldehyde. As the buffer for the preparation of the fixation solution of formaldehyde, it is preferable to use a phosphate buffer or a phosphate buffered physiological solution (1x PBS). For the preparation of the 1 to 8 % (weight/vol.) solution of formaldehyde, it is also preferable to use a methanol stabilized solution of formaldehyde. Another suitable and rapid method of fixation is the incubation of samples containing cells in a 50 to 100 % solution of ethanol or methanol preferably in water or in a aqueous buffered solution, e.g. using a phosphate buffer or 1x PBS. The period of fixation or the temperature of the fixation solutions is not important for the described method of determining the DNA. For instance, a period of incubation already from 30 seconds and a temperature preferably from 4 °C to 30 °C is sufficient for formaldehyde fixation. For ethanol fixation, it is preferable to use a period of fixation longer than 10 minutes. The samples could be stored in an ethanol or methanol fixation for a period of several months to several years, preferably at low temperatures, for example at -20 °C to -10 °C. The method can be used also for dried or non-fixed cells.

In one preferred embodiment of the present invention, the incubation of the sample with at least one fluorescent substance in step (i) is conducted at a temperature of 0 to 45 °C for a period longer than 2 minutes; the washing of the sample with the aqueous washing solution in step (ii) is performed preferably at a temperature of 0 to 45 °C for a period longer than 10 minutes, wherein in the course of this time the washing solution is changed at least 2x for a new one, more preferably this step is conducted for a period of 15 to 60 minutes and the washing solution is changed for a new one at least 3x; the elution of the fluorescent substance from the DNA in step (iii) is performed preferably at a temperature of 0 to 45 °C for a period longer than 2 minutes.

Our experiments showed that in the case of DAPI the optimal concentration of SDS and LiDS is 1 - 2 % (weight/vol.) in the majority of cases in terms of the speed of elution and the signal achieved. This value was similar in the use of DAPI and sarkosyl. For Hoechst and SDS or LiDS, the optimal concentrations are between 5 - 10 % (weight/vol.) of SDS or LiDS. In the case of Hoechst and sarkosyl, these concentrations are minimally 0.6 % (weight/vol.) of sarkosyl, but the 0.6 % (weight/vol.) concentration of sarkosyl was not sufficient in some cases for the quick washing out of DAPI and/or Hoechst from DNA. Generally, the use of SDS and LiDS was more preferable than the use of sarkosyl. The results also showed that the measured signal of DAPI in SDS and LiDS was generally significantly higher than the measured signal of Hoechst. We also tested the possibility to use mutual mixtures of the elution substances SDS, LiDS and sarkosyl. These mixtures also led to the washing out of DAPI and/or Hoechst and to the increase of fluorescence.

For accelerating the elution, it is optimal to agitate the samples on a laboratory shaker, preferably at 300 to 600 rpm, during the elution process. The admixtures of ethanol, methanol, isopropanol or the components of the buffers, e.g. phosphates or Tris, do not have a significant influence on the elution or subsequent fluorescence intensity. During the development it was shown that in contradiction to the earlier published results (J. Immunol. Methods, 162, 41-45), ethanol did not lead to elution of DAPI or Hoechst. It is clear from the measured data that the signal provided by DAPI is independent of pH in the area of pH 6 - 8. In the case of Hoechst, the optimum value is a pH of 6.5 - 7.5. At the same time, it was clear that the fluorescent signal was stable at least for a period of several weeks, what is significant both from the perspective of the possibility of the prospective transport of the sample to a suitably equipped laboratory, and from the perspective of the possibility of repeated measurement and the preservation of the samples for subsequent evaluation.

In one preferred embodiment of the present invention, if the samples are cells or tissues, then before the step (i) or concurrently with the step (i) or before the step (ii) or before the step (iii), incubation of the sample in an aqueous solution containing monovalent copper ions is performed. Although the incubation in an aqueous solution containing monovalent copper ions is possible to conduct at the same time as the incubation with DAPI and/or Hoechst (simultaneously with the step (i)) or any time before the elution step (iii) (before the step (i) or before the step (ii) or before the step (iii)), its effect is the highest when used before the incubation with DAPI and/or Hoechst (before step (i)). This step increases the signal and, at the same time, this step can be used instead of the fixation step. When a solution of monovalent copper and populations of adherent cells were used, there was a stabilization of the cells to the support to the extent that it was not necessary to fix the cells so as not to release them from the support by the elution substances. At the same time, this step significantly reduced, the viscosity of this solution due to DNA cleavage in cases, where DNA from cell-containing samples was released into the solution (*PLoS One,* 7, e52584), which facilitated the manipulation with the solution in its prospective transfer to another vessel intended for a measurement. The incubation in the aqueous solution of monovalent copper did not influence the linearity of the measurement. Solutions containing monovalent copper, including those listed in Patent No. CZ 303230, were tested, such as aqueous solution of copper (II) sulphate and aqueous solution of sodium ascorbate or aqueous solution of copper (II) sulphate and aqueous solution of hydrazine or aqueous solution of copper (II) sulphate and aqueous solution of tris(2-carboxyethyl)phosphine (TCEP) or aqueous solution of copper (II) sulphate and aqueous solution of hydroquinone or aqueous solution of copper (II) chloride and aqueous solution of sodium ascorbate or aqueous solution of copper (II) chloride and aqueous solution of hydrazine or aqueous solution of copper (II) chloride and aqueous solution of TCEP or aqueous solution of copper (II) chloride and aqueous solution of hydroquinone or aqueous solution of copper (II) nitrate and aqueous solution of sodium ascorbate or aqueous solution of copper (II) nitrate and aqueous solution of hydrazine or aqueous solution of copper (II) nitrate and aqueous solution of TCEP or aqueous solution of copper(II) nitrate and aqueous solution of hydroquinone or aqueous solution of copper (II) acetate and aqueous solution of sodium ascorbate or aqueous solution of copper (II) acetate and aqueous solution of hydrazine or aqueous solution of copper (II) acetate and aqueous solution of TCEP or aqueous solution of copper (II) acetate and aqueous solution of hydroquinone or aqueous solution of copper (II) bromide and aqueous solution of sodium ascorbate or aqueous solution of copper (II) bromide and aqueous solution of hydrazine or aqueous solution of copper (II) bromide and aqueous solution of TCEP or aqueous solution of copper (II) bromide and aqueous solution of hydroquinone or aqueous solution of tetrakis(acetonitrile)copper(I) hexafluorophosphate ([Cu(CH₃CN)₄]PF₆) or aqueous solution of tetrakis(acetonitrile)copper(I) tetrafluoroborate ([Cu(CH₃CN)₄]BF₄).
The use of an aqueous solution prepared by mixing an aqueous solution of hydroquinone and an aqueous solution of copper (II) sulphate was shown to be the most preferable. Unlike the relatively abundantly used system based on mixing an aqueous solution of copper sulphate and an aqueous solution of sodium ascorbate, this system did not require the presence of other substances preventing the formation of a visible precipitate. The final concentration of copper sulphate in the solution, in which monovalent copper formed, was preferably 0.1 - 50 mmol.l⁻¹ at the moment of the addition and the final concentration of hydrochinon was preferably 0.5 - 100 mmol.l⁻¹ at the moment of addition. For the preparation of a solution of monovalent copper, it is preferable first to prepare a stock aqueous solution of hydroquinone and a stock aqueous solution of copper sulphate. These two solutions are mixed just before the use of the solution of monovalent copper. Preferably, the stock aqueous solution of copper sulphate is prepared in concentrations of 4 - 200 mmol.l⁻¹ and the stock aqueous solution of hydroquinone in concentrations of 20 - 200 mmol.l⁻¹. The incubation of the sample in the solution of monovalent copper preferably takes place for a period of 1 minute to 1 hour at temperatures of 0 to 45 °C.

Because of the fact that all of the fluorescent substances are in the elution solution at the end of the described method, it is possible to perform a measurement of the fluorescence either directly in the vessel, wherein the fluorescent substances were eluted, or, after transfer of the solution, in another vessel. This is preferable in all of the cases when the samples are in vessels, which are not suitable for measuring fluorescence. At the same time, this method provides a homogeneous signal in the whole volume of the elution solution, which significantly increases the reliability of the results obtained and reduces the need for repeated measurements. The storage of these elution solutions is also possible for a longer period of time, e.g. for the period of several weeks and their subsequent measurement.

The results showed that the signal provided by DNA increased linearly with increasing concentration of DNA, and this linearity was maintained in a broad band of tested concentrations of SDS, LiDS and sarkosyl, preferably from 0.1 % to 20 % (weight/vol.). The method provides relative data. After calibration, for example, in the case of DNA determination using absorbance at 260 nm, or in the case of determination of amount of cells by means of a haemocytometer, it is possible to determine also the absolute amount of DNA or the absolute number of cells. According to our findings, the concurrent presence of DNA, RNA, proteins and sugars at the moment of measurement does not interfere with DAPI and Hoechst signals in the elution solutions and does not affect the linearity of the measurement. Although DAPI and Hoechst bind to RNA, which is in the case of Hoechst primarily interaction with double-stranded RNA segments, it is not necessary to remove RNA during, or just before applying the method of determining the number of cells. This is due to a similar RNA content in differently growing populations. If necessary, it is possible to eliminate the content of RNA entirely or significantly using incubation in an aqueous solution of monovalent copper and/or by the use of RNases.

For determining the background, which is caused by the binding of the fluorescent substances to the samples and other parts, which were used in the course of the application of the method, it is preferable to perform the entire method at the same time in a vessel without samples containing DNA e.g. without anchored DNA or without cells, and after measuring the signal to subtract this signal from the signal that was measured in the samples containing DNA.

Preferably, the method is also usable in the case when other cellular components are determined using probes labelled with fluorochromes (fluorescent probes), e.g. proteins and/or RNA and/or specific DNA sequences and/or lipids and/or sugars and/or substances, which are incorporated into cells and it is necessary to relate their signal to the number of cells. Generally, any fluorochrome can be used as the fluorescent probe, if it has affinity towards the determined cellular component. These fluorescent probes could be the fluorochrome itself or its conjugate with an antibody, which has affinity towards the determined cellular component (it is capable of conjugation to the determined cellular component). It is also possible to use fluorochrome-labelled nucleotides, sugars or amino acids. The fluorescent probes can further be for example lectins or sequences of DNA or RNA labelled with fluorochrome or azide fluorochromes or fluorochromes containing groups which reacts with the determined cellular component. Generally, it is preferable to use fluorochromes with the maximal emission wavelength higher than 470 nm or lower than 445 nm. The examples of fluorochromes are for example carboxyfluorescein (FAM) or fluorescein isothiocyanate (FITC) or coumarin (coumarin 343) or cyanine (Cyanine3 (Cy3), or Cyanine3.5 (Cy3.5), or Cyanine5 (Cy5) or Cyanine5.5 (Cy5.5) or Cyanine7 (Cy7) or Cyanine7.5 (Cy7.5)) or phenylethynylpyrene (PEP) or rhodamine (rhodamine 101 (ROX)) or tetramethylrhodamine 5-carboxamido-(6-azidohexanyl) (TAMRA) or 6-carboxamido-(6-azidohexanyl) (Oregon Green). The method is also usable in the case when it is so-called indirect immunolabelling, when a non-fluorescent probe reacting with the detected molecule is first used or several consecutive non-fluorescent probes and only against the last of these probes a fluorescent probe reacting with this probe is used. The typical example is, e.g., the detection of cellular proteins using the first antibody, which first binds to the protein and the second antibody is used subsequently that is conjugated with fluorochrome and reacts with the first antibody.
The method is also usable in cases, wherein it is necessary to relate the amount of the fluorescently-labelled DNA and/or fluorescently-labelled RNA and/or fluorescently-labelled proteins in cells to the number of cells. The example is cells expressing fluorescent proteins and/or containing DNA and/or RNA labelled with nucleosides conjugated with a fluorochrome.

An example of the substances, which are determined after their incorporation into the DNA of the cells, is 5-ethynyl-2'-deoxyuridine (EdU) or 5-bromo-2'-deoxyuridine (BrdU) or 5-chloro-2'-deoxyuridine (CldU) or 5-iodo-2'-deoxyuridine (IdU). EdU, BrdU, CldU and IdU are incorporated into cellular DNA and it is possible, on the basis on the amount of the incorporated EdU and/or BrdU and/or CldU and/or IdU, to decide about the replication activity of the cells. In all of these cases, it is preferable to determine the DNA at the same time and subsequently the number of cells using the described method. The described method allows to relate the signal intensity of the fluorescent probes to the number of cells. By dividing of the signal of the fluorescent probes by the signal of DAPI and/or Hoechst in the elution solution, the influence of the number of cells on the signal provided by the fluorescent probes is thus removed. After the calibration of the signal of DAPI and/or Hoechst to the actual number of cells, e.g. using a haemocytometer, it is then possible to determine also the average signal in the cell.

EdU, BrdU, CldU and IdU interfere with Hoechst and reduce their fluorescence, without reducing the binding capacity of fluorescent substances to DNA. If an estimate of the number of cells by a simple measurement of the fluorescence of the bound fluorescent substances Hoechst to the DNA is used in these cases, it leads to an underestimation of the number of cells. Even higher interference may occur when certain azide fluorochromes are bound to EdU. In this case, interference occurs even in the case that not only Hoechst but also DAPI is used. For instance, in the usage of FAM azide, a rapid drop of the fluorescence of DAPI occurs, and, subsequently, it leads to an underestimation of the number of cells again. In all of these cases, it is suitable to use the method we have described for determining the number of cells, when the signal of DAPI and/or Hoechst is determined in the elution solution. The described method then allows not only the precise determination of the number of cells without a relation to the content of the substances of the type EdU or BrdU or CldU or IdU, but also subsequently makes it possible to relate the signal of the fluorescent probes used to the number of cells.

In the course of the detection of the cellular components and/or incorporated substances using fluorescent probes, incubation with fluorescent substances (DAPI and/or Hoechst) is performed, followed by washing using washing solutions and then incubation in the elution solution is performed. If fluorescent probes non-covalently bound to the cellular components being detected are used, the addition of elution substances usually leads to the passing of the fluorescent probe into the solution. In some cases, addition of the elution substances leads to lysis of the cells. That is particularly in the case, when a solution containing agents forming covalent bonds between the various cellular components, e.g. proteins, was not used for the fixation of the cells. An example of an agent forming covalent bonds is formaldehyde. The usage of a solution of monovalent copper also leads to stabilization of the cells, which prevents their lysis in a solution of an elution substance. Generally, it is preferable to release fluorescent probes into a solution either by the action of the elution solution or by the evocation of cellular lysis.

In one embodiment of the present invention, if the sample is cells or tissues, then between the steps (ii) and (iii) or simultaneously with the step (iii) the sample is incubated with proteinase K. In this embodiment, the fluorescent probes strongly bound, for example covalently, to the cellular components being detected are used, and the elution solutions do not lead to the transfer of the cellular content to the elution solution,. It is, for instance, a case when EdU is used and in some step formaldehyde and/or a solution of monovalent copper is used. In these cases, it is usually not possible to release the fluorescent probes into the elution solution using elution substances and to ensure that the elution solution would contain not only DAPI and/or Hoechst, but also the fluorescent probes. Proteinase K can be applied in the elution solution. Preferably, the elution solutions are added to the sample, after its incubation with proteinase K. The elution substances are also preferably added to the solution, which contains the cells influenced by proteinase K after its transfer to a new vessel. This leads to a significant reduction of the background. Subsequently, the fluorescence of the fluorescent probes and DAPI and/or Hoechst in the elution solution is measured. The signal provided by the bound fluorescent probes is divided by the signal of DAPI and/or Hoechst. The signal measured in the control samples without DNA is before this step preferably subtracted from the signal of DAPI and/or Hoechst. It is also possible to measure the signal provided by fluorescent probes just before the addition of the elution substances.

It is also preferable to use proteinase K in the case of determining the number of cells in all cases, when the surfaces of the vessels strongly bind DAPI and Hoechst and the application of the elution solutions would lead to the washing out of DAPI and/or Hoechst from these surfaces and thus would lead to an increase of the background. For this purpose, incubation with proteinase K is used before the elution step, when its activity leads to the transfer of the cellular content to the solution. A part or all of the solution is then transferred to another vessel and only then the elution solution is added. This lowers the background.

Generally, it is always preferable to remove the samples from the vessel, where the incubation with fluorescent substances took place, before adding the elution solution.

The concentration of proteinase K is preferably from 0.05 to 0.2 mg.ml⁻¹, wherein proteinase K is preferably diluted in 50 mmol.l⁻¹ of an aqueous solution Tris-HCl, pH = 8. The samples are incubated in a solution of proteinase K for 5 to 120 minutes, preferably at a temperature of 37 °C. The samples are also preferably agitated on a laboratory shaker during the incubation.

Another use of the described method of determining the amount of DNA and subsequently also the number of cells is its use for determining the replication activity. In these cases, the samples containing cells are first incubated in a cultivation medium with 0.1 to 100 µmol.l⁻¹ BrdU and/or IdU and/or CldU and/or EdU preferably for a period equal to at least 1/3 of the cell cycle of the tested cellular population. Subsequently, the incubation with Hoechst is conducted just like in the case of determining the amount of DNA. This incubation can take place still in the medium or, preferably, after fixation of the samples, e.g., with ethanol. It is, however, possible to use also other methods of fixation or to dry the samples containing cells or to incubate the samples containing cells in an aqueous solution, which contains monovalent copper ions just like in the method for determining the amount of DNA in samples containing cells. Washing with washing solutions is further performed just like in the method for determining the amount of DNA. Incubation with proteinase K is further carried out; this incubation takes place just like in the case, when proteinase K is used for determining the DNA in samples containing cells. Subsequently, the fluorescent signal of Hoechst is measured. Since the incorporation of the mentioned nucleosides strongly suppress the fluorescence of Hoechst, the signal decreases with the increasing degree of the incorporation of these nucleosides. Nevertheless, the signal of Hoechst also includes in itself the influence of a different number of cells. The signal of Hoechst is subsequently preferably reduced by the signal measured in the control samples without DNA. Then, the elution solution is added to the samples. The addition of the elution solution leads to the elution (washing out) of the molecules of Hoechst bound to DNA into the elution solution. The fluorescent signal of Hoechst in the elution solution is then measured. This signal is proportional to the number of cells and does not depend on the amount of the incorporated nucleosides used. Preferably, the signal of Hoechst is subsequently reduced by the signal measured in the control samples without DNA. Since the mentioned nucleosides do not suppress the binding of Hoechst to DNA, but only their fluorescence, the signal measured is proportional to the number of cells. By dividing the signal measured after the action of proteinase K by the signal measured after the application of the elution solutions, the value reached precisely reflects the replication activity without any dependence on the number of cells. The higher the signal is, the lower replication activity it signalizes.

The described method of determining DNA is fast and thanks to the low material demands and the fact that the measurement can take place in another vessel than the sample of DNA is found e.g. a sample containing cells, it is suitable for the processing of high numbers of samples and/or large samples. The method also allows processing of the cells in suspension or samples of tissues. An indisputable advantage of the entire method is the possibility of some embodiments of repeated use of the sample with DNA e.g. the sample containing cells for further processing without there being a loss of DNA or the ability of further reactions of the DNA. Another advantage is the fact that in the case of the simultaneous detection of replication activity using halogen derivatives of 2'-deoxyuridine or EdU there is a drop of the signal provided by Hoechst and/or DAPI, which is not caused by the lower binding capacity of the fluorescent substances to DNA, but by the drop of the fluorescence of the fluorescent substances bound to the labelled DNA. The elution therefore eliminates the decrease of the fluorescence of the fluorescent substances used. As it is clear, this effect is especially significant in the case, when azide fluorochrome is used for the detection of EdU using the so-called "click reaction". At the same time, the use of fluorochromes is advantageous from the perspective of the speed of the detection of EdU. The described technology entirely removes the problems with the detection of EdU and concurrent quantification of the number of cells using DAPI and/or Hoechst. As it was shown, the technology is also usable in the detection of replication activity using BrdU, CldU, IdU and EdU, when the decrease of the fluorescence of Hoechst in a bond to DNA is used, which contains the mentioned nucleosides.

One of the preferred embodiments of determining the amount of DNA is based on the incubation of the samples containing DNA anchored on the surfaces or particles with an aqueous, preferably buffered, solution of DAPI and/or Hoechst, subsequent washing in an aqueous, preferably buffered washing solution containing preferable a detergent e.g. Tween 20, and elution using aqueous, preferably buffered elution solution containing SDS and/or LiDS.

In one preferred embodiment in terms of price, the strength of the signal and the speed in the case of samples containing cells, the samples are fixed with ethanol or methanol, preferably 50 to 100 % (vol./vol.) solution of ethanol or methanol in water or in a phosphate buffer or 1x PBS, incubated in an aqueous, preferably buffered solution of DAPI and/or Hoechst, washed in an aqueous, preferably buffered washing solution, which preferably contains Tween 20 and subsequently eluted in an aqueous, preferably buffered elution solution containing SDS and/or LiDS.

In another embodiment, the fixation solution of ethanol or methanol contains DAPI and/or Hoechst.

In the case of the adherent cell cultures, when it is necessary for the cells to be kept at the cultivation surface for the entire time of the method, it is preferable when the cells are fixed in a solution of formaldehyde, preferably in a phosphate buffer or 1x PBS, or the cells are fixed by ethanol or methanol, preferably a 50 to 100 % (vol./vol.) solution of ethanol or methanol in water or a phosphate buffer or 1x PBS. An incubation in an aqueous solution of monovalent copper is preferably performed subsequently or just before the fixation. It is also possible to fix the cells only with a solution of monovalent copper or e.g. by simple drying. The solution of monovalent copper is preferably prepared from an aqueous solution of hydroquinone and an aqueous solution of copper (II) sulphate. The solution of monovalent copper is preferably used also in all of the cases when it is necessary to increase the signal provided by DAPI and/or Hoechst.

The determination of DNA is performed in several steps, which may but do not have to follow closely one after another:
(i) The samples are incubated in a solution, preferably an aqueous and also preferably a buffered solution, of at least one fluorescent substance selected from the group consisted of DAPI, Hoechst 33258, Hoechst 33342 and Hoechst 34580, which, in the course of this incubation, binds to the DNA present in the sample. For the buffering it is preferable to use a phosphate buffer or 1x PBS or buffers containing Tris or HEPES. In the case of the determination of DNA in samples containing cells, the fluorescent substance is preferably dissolved in a cultivation medium.
(ii) The samples are washed with a washing aqueous solution, preferably with a solution of a buffer, the pH of which is also preferably from 6 to 8. The washing solution further preferably contains at least one detergent. The buffer is preferably a phosphate buffer or 1x PBS.
(iii) Fluorescent substances bound to DNA in a sample are eluted from the DNA with a elution aqueous, preferably buffered, solution of at least one of the substances selected from the group of SDS, LiDS and sarkosyl. The buffer preferably contains Tris.
(iv) The amount of DNA in the elution solution from the previous step is determined by measuring the fluorescent signal of the fluorescent substances used in step (i).

In one preferred embodiment, the washing solution contains at least one detergent, which is preferably selected from the group comprising Tween 20, Tween 80, Triton X-100, saponins, Nonidet P-40, SDS, LiDS, sarkosyl, wherein the concentration of the detergent in the washing solution is preferably in the range from 0.01 % (vol./vol.) to 10 % (vol./vol.), preferably from 0.05 to 5 % (vol./vol.), and in the case of the use of SDS and/or LiDS and/or sarkosyl as a detergent, their concentration is at most 0.05 % (weight/vol.) and at the same time the used concentrations of the these substances in the washing solution are preferably at least 10x lower than their concentrations used in the elution solution.

In one preferred embodiment, the incubation of the sample with DAPI and/or Hoechst is performed at a temperature from 0 °C to 45 °C for a period longer than 2 minutes.

In another preferred embodiment, the washing of the sample is performed by a washing aqueous solution preferably at a temperature from 0 °C to 45 °C for a period longer than 10 minutes, wherein in the course of this time the washing solution is changed for new one at least 2x, more preferably this step is performed for a period of 15 to 60 minutes and the washing solution is changed for a new one at least 3x.

In yet another preferred embodiment, the elution of the fluorescent substance from DNA is performed preferably at a temperature of 0 °C to 45 °C for a period longer than 2 minutes.

In another preferred embodiment, in the case that the sample is cells or tissue, between the washing of the sample in a washing solution and the elution of DAPI and/or Hoechst from the DNA or at the same time with the elution of DAPI and/or Hoechst from the DNA in the elution solution, the sample is incubated with proteinase K.

In another preferred embodiment, the background correction of the signal provided by DAPI and/or Hoechst in the case of cells cultivated in Petri dishes, or cultivation flasks, or 12-, or 24-, or 48-, 96- well plates or on glasses is performed by measuring the signal from Petri dishes, or cultivation flasks, or 12-, or 24-, or 48-, 96- well plates, or glasses without cells. In the case of the performance of several repetitions, it is preferable to set the average signal in wells from Petri dishes, or cultivation flasks, or 12-, or 24-, or 48-, 96- well plates, or glasses without cells and it is further worked with the average values of these signals. This signal is subsequently subtracted from the individual signals of the samples with cells. The relative values of these corrected DAPI and/or Hoechst signals calculated for the individual samples then reflect the relative values of the number of cells in the individual samples. In the case that it is necessary to perform a precise determination of the cells in the particular samples, the number of cells is simultaneously determined in some samples preferably using a microscope and CCD camera and subsequently the average signal of DAPI and/or Hoechst in one cell is calculated. The corrected signals of DAPI and/or Hoechst provided by the individual samples are then divided by this signal and the number of cells in the individual samples is determined. Alternatively, the samples of cells containing a known number of cells are used, e.g. serially diluted samples of cells which are processed according to the described approach, then the calibration curve is calculated and subsequently the number of cells in the individual samples is determined with its help.

The object of the present invention is further a method of determining the amount of 5-ethynyl-2'-deoxyuridine and/or 5-bromo-2'-deoxyuridine and/or 5-chloro-2'-deoxyuridine and/or 5-iodo-2'-deoxyuridine incorporated into DNA and/or the content of proteins and/or the amount of RNA and/or the amount of specific DNA sequences and/or the amount of sugars and/or the amount of lipids and/or the amount of fluorescently-labelled DNA and/or fluorescently-labelled RNA and/or fluorescently-labelled proteins in cells and/or tissues using the method according to the present invention, wherein the incubation of cells and/or tissues with a fluorescent probe is performed, in the course of which the fluorescent probe binds to the 5-ethynyl-2'-deoxyuridine and/or 5-bromo-2'-deoxyuridine and/or 5-chloro-2'-deoxyuridine and/or 5-iodo-2'-deoxyuridine incorporated into DNA and/or to the proteins and/or RNA and/or specific DNA sequence and/or sugar and/or lipid and/or fluorescently-labelled DNA and/or fluorescently-labelled RNA and/or fluorescently-labelled proteins; wherein the fluorescent probe is compounds selected from the group of fluorochromes and fluorochromes conjugated with antibodies, lectins, RNA, DNA or azide functional groups,
wherein before, in the course of or after the reaction with the fluorescent probe, the steps (i) and (ii) of the method according to the present invention are performed,
and, subsequently, after the reaction with the fluorescent probe and after performing the steps (i) and (ii) of the method according to any one of the preceding claims, the step (iii) of the method according to any one of the preceding claims is performed;
subsequently, the fluorescence signal provided by the fluorescent probes and/or by the fluorescently-labelled DNA and/or fluorescently-labelled RNA and/or fluorescently-labelled proteins is measured, as well as the fluorescence signal provided by the fluorescent substance selected from the group consisted of 4',6-diamidino-2-phenylindole, 2'-(4-hydroxyphenyl)-5-(4-methyl-1 -piperazinyl)-2,5'-bi-1H-benzimidazole, 2'-(4-ethoxyphenyl)-5-(4-methyl-1-piperazinyl)-2,5'-bi-1H-benzimidazole and N,N-dimethyl-4-[5-(4-methyl-1-piperazinyl)[2,5'-bi-1H-benzimidazole]-2'-yl]benzenamine is measured;
thereafter, the signal provided by the fluorescent probes and/or by the fluorescently-labelled DNA and/or fluorescently-labelled RNA and/or fluorescently-labelled proteins is divided by the fluorescent signal provided by the fluorescent substance, corresponding to the number of cells in the sample, and the amount of 5-ethynyl-2'-deoxyuridine and/or 5-bromo-2'-deoxyuridine and/or 5-chloro-2'-deoxyuridine and/or 5-iodo-2'-deoxyuridine incorporated into DNA and/or the content of proteins and/or the amount of RNA and/or the amount of specific DNA sequences and/or the amount of sugars and/or the amount of lipids and/or the amount of fluorescently-labelled DNA and/or fluorescently-labelled RNA and/or fluorescently-labelled proteins in cells or tissues of the sample is determined. This method of determining the amount of EdU and/or BrdU and/or CldU and/or IdU incorporated into DNA and/or proteins and/or RNA and/or specific DNA sequences and/or of sugars and/or of lipids in cells and/or tissues is based on the detection methods based on the specific direct or indirect binding of the fluorescent probes with the above-mentioned substances and the measured signal provided by the fluorescent probes is related to the signal reflecting the number of cells measured using the described method of determining the number of cells. Following the incubation of the sample of cells or tissues with EdU and/or BrdU and/or CldU and/or IdU, a reaction with fluorescent probe -- azide fluorochrome (if the amount of EdU incorporated into DNA is determined), or with an antibody labelled with a fluorochrome reacting with the used nucleoside (if the amount of EdU and/or BrdU and/or CldU and/or IdU incorporated into DNA is determined) is performed. The examples of azide fluorochromes are carboxyfluorescein (FAM) or fluorescein isothiocyanate (FITC) or coumarin 343 or fluorochromes from the group of polymethines(particularly cyanines such as Cyanine3 (Cy3) or Cyanine3.5 (Cy3.5) or Cyanine5 (Cy5) or Cyanine5.5 (Cy5.5) or Cyanine7 (Cy7) or Cyanine7.5 (Cy7.5)), or phenylethynylpyrene (PEP) or fluorochromes containing fluorone structure unit, particularly fluorescein and rhodamine, for example rhodamine 101 (ROX) or tetramethylrhodamine 5-carboxamido-(6-azidohexanyl) (TAMRA) or 6-carboxamido-(6-azidohexanyl) (Oregon Green); or a reaction with a primary and subsequently a secondary antibody is performed, whereas the primary antibody reacts with the nucleoside being determined, and the secondary antibody reacts with the primary antibody, wherein the secondary antibody is conjugated with the fluorochrome. The examples of fluorochromes are FAM, FITC, Cy3, Cy3.5, Cy5, Cy5.5, Cy7, Cy7.5, TAMRA, in the case of determining proteins or RNA or DNA or specific DNA sequences or sugars or lipids, the sample of cells or tissues at first undergoes the reaction of the molecule being determined with the probe, which can be e.g. an antibody or RNA or DNA or lectin, and which can be labelled with a fluorochrome or is without a fluorochrome. In the latter case, a subsequent reaction with a secondary probe follows, wherein the primary probe (e.g. antibody) reacts with the molecule being determined, and the secondary probe (e.g. antibody reacting with the primary probe) reacts with the primary probe, and whereas the secondary probe is conjugated with a fluorochrome An example of fluorochrome is FAM, FITC, Cy3, Cy3.5, Cy5, Cy5.5, Cy7, Cy7.5, TAMRA, Texas Red (sulphforhodamine acid chloride); whereas during these steps or after they are finished, the steps (i), (ii) and (iii) of the method according to the present invention are performed; subsequently, the fluorescence signal provided by fluorescent probes, bound during the detection process to EdU and/or BrdU and/or CldU and/or IdU incorporated into DNA, and/or to proteins and/or to RNA and/or to the specific DNA sequences and/or to sugars and/or to lipids is measured and the signal provided by the fluorescent substance selected from a group consisting of DAPI and/or Hoechst is also measured; subsequently, the fluorescence signal provided by the fluorescence substance is compared to the signal provided by the fluorescent probes (the signal provided by the fluorescence substance is divided by the signal provided by DAPI and/or Hoechst). Preferably, the fluorescence signal provided by DAPI and/or Hoechst is reduced by the signal from control samples not containing cells or tissues, and, whereas preferably, before or during the incubation of the sample in the elution solution, the sample is incubated with proteinase K, and whereas, preferably, before the incubation of the sample with DAPI and/or Hoechst, the incubation of the sample with monovalent copper ions solution is performed.
The procedure is analogical if it is necessary to relate the signal provided by fluorescently-labelled proteins, DNA or RNA in cells to the number of cells, it only differs in the fact that the detection of these proteins, DNA or RNA is not performed using fluorescent probes.

In one preferred embodiment of determination of the amount of EdU incorporated into DNA and of the derived replication activity using azide fluorochromes reacting with EdU, where the fluorochrome can be e.g. FAM or FITC or coumarin 343 or Cy3 or Cy3.5 or Cy5 or Cy5.5 or Cy7 or Cy7.5 or PEP or ROX or TAMRA or Oregon Green, the signal provided by the azide fluorochrome reacting with EdU is divided by the corrected signal provided by DAPI and/or Hoechst and/or the calculated number of cells. In the case of performance of several repetitions, the average signal provided by azide fluorochrome reacting with EdU is determined and the average corrected signal provided by DAPI and/or Hoechst and/or average number of cells in these samples is determined and only then is the average signal provided by azide fluorochrome reacting with EdU is subsequently divided by the corrected signal provided by DAPI and/or Hoechst and/or the average number of cells. The calculated value is then used for a comparison of the content of EdU and mutual replication activity in various samples. This method is suitable primarily in the case when the used azide fluorochromes reacting with EdU do not bind strongly on the cellular structures and/or on the Petri dishes, or cultivation flasks or 12-, or 24-, or 48-, 96-well plates, or glasses and/or they are easily removed by washing.

In the embodiments, when it is necessary to perform a correction on the background in the case of determining the amount of EdU in cellular DNA and the derived replication activity, it is possible to proceed e.g. in these two ways:
a) First, the signal provided by azide fluorochrome is measured in samples with cells, which were cultivated with EdU and at the same time in the samples of cells, which were not cultivated with EdU. Subsequently, the signal in the samples cultivated without EdU and in samples cultivated with EdU is divided by the value of the corrected signal of DAPI and/or Hoechst and/or the calculated number of cells. In the case of an embodiment, when several repetitions of the samples of cells cultivated with or without EdU is performed, the average signal of the azide fluorochrome reacting with EdU is preferably calculated first and also the average corrected signal provided by DAPI and/or Hoechst and/or the average number of cells from these repetitions and this is used in these and the subsequent calculations. This method is especially suitable in the case of cells, which were cultivated without EdU. Subsequently, the signal provided by azide fluorochrome reacting with EdU is divided by the corrected signal provided by DAPI and/or Hoechst and/or the calculated number of cells. The calculated values for samples with cells cultivated without EdU are deducted from the values calculated for samples with cells cultivated with EdU. The calculated values are, if it is necessary and does not arise directly from the previous calculation, averaged for the individual repetition. The calculated values are used for the comparison of the content of EdU and mutual replication activity in various samples. This method is suitable primarily in the case when the main part of the background is derived from the strong bond of the azide fluorochrome on the cellular components and only a small part of the background is caused by its bond to the Petri dishes, or cultivation bottles, or 12-, or 24-, or 48-, 96-well plates, or glasses.
b) In the second case, the signal provided by azide fluorochrome in samples with cells that are cultivated with EdU and simultaneously in the samples of cells that are not cultivated with EdU, is measured first. Moreover, the signal in samples without cells is measured. The signal in samples without cells is subsequently subtracted from the signal provided by azide fluorochrome reacting with EdU in samples with cells. In the case that the samples without cells were processed in several repetitions, it is preferable to use the average of the measured values of the signals for the calculations. Subsequently, the calculated values of the signals in the samples cultivated without EdU and in the samples cultivated with EdU are divided by the value of the corrected signal of DAPI and/or Hoechst and/or the calculated number of cells. In the case of an embodiment, when several repetitions of the samples of the cells cultivated with or without EdU is performed, it is preferable to calculate first the average signal of the azide fluorochrome reacting with EdU and also the average corrected signal provided by DAPI and/or Hoechst and/or the average number of cells from these repetitions and this is used in these and the subsequent calculations. This method is especially suitable in the case of cells, which are cultivated without EdU. Subsequently the signal provided by azide fluorochrome reacting with EdU is divided by the corrected signal provided by DAPI and/or Hoechst and/or the calculated number of cells. The calculated values for samples with cells cultivated without EdU are deducted from the values calculated for samples with cells cultivated with EdU. The calculated values are, if it is necessary and does not arise directly from the previous calculation, averaged for the individual repetitions. The calculated values are used for the comparison of the content of EdU and the mutual replication activity in various samples. This method is suitable primarily in the case when a significant amount of the background is derived from strong bond of azide fluorochrome to Petri dishes, or cultivation flasks, or 12-, or 24-, or 48-, 96-well plates, or glasses.

In another preferred embodiment, the amount of protein in the cells is determined using primary antibody, which reacts with this protein, and secondary antibody reacting with the primary antibody, wherein the secondary antibody is conjugated with a fluorochrome, e.g. FAM or FITC or Cy3 or Cy3.5 or Cy5 or Cy5.5 or Cy7 or Cy7.5 or TAMRA or Texas Red, by dividing the signal provided by the antibody conjugated with the fluorochrome by the corrected signal provided by DAPI and/or Hoechst and/or the calculated number of cells. In the case of the performance of several repetitions, the average signal provided by the antibody conjugated with the fluorochrome and the average corrected signal provided by DAPI and/or Hoechst and/or the average number of cells in these samples is determined in some embodiments, and only then the average signal provided by the antibody conjugated with fluorochrome is divided by the average corrected signal provided by DAPI and/or Hoechst and/or the average number of cells. The calculated value is then used for a comparison of the mutual replication activity in various samples. This method is suitable primarily in the case when the used antibodies and/or the antibodies conjugated with the fluorochrome do not bind strongly to the cellular structures and/or on a Petri dish, or cultivation flasks, or 12-, or 24-, or 48-, 96-well plate, or glass and/or it is possible to remove them easily by washing.

In the embodiments, when the amount of protein in cells is determined using a primary antibody, which reacts with this protein and a secondary antibody reacting with the primary antibody, wherein the secondary antibody is conjugated with a fluorochrome and it is necessary to perform a correction on the background, it is possible to continue e.g. in these two ways:
a) First, the signal provided by the secondary antibody conjugated with fluorochrome in the samples with cells, which were incubated both with primary and secondary antibody and in the samples with cells, wherein the incubation with the primary antibody was omitted, is measured. Subsequently, the signal in the samples with cells, which were incubated both with primary and secondary antibody and in the samples with cells, wherein the incubation with the primary antibody was omitted, is divided by the value of the corrected signal of DAPI and/or Hoechst and/or calculated number of cells. In the embodiment, when several repetitions of the samples with cells incubated or non-incubated with the primary antibody were performed, the average signal provided by secondary antibody conjugated with fluorochrome and also the average corrected the signal provided by DAPI and/or Hoechst and/or the average number of cells from these repetitions is preferably calculated first, and this average is then used in these and any subsequent calculations. This method is especially suitable in the case of cells, which were not incubated with the primary antibody. Subsequently, the signal provided by secondary antibody conjugated with a fluorochrome is divided by the corrected signal provided by DAPI and/or Hoechst and/or the calculated number of cells. The calculated values for samples with cells, which were not incubated with the primary antibody, are deducted from the values calculated for samples with cells incubated with the primary antibody. The calculated values are, if it is necessary and it does not arise directly from the previous calculation, averaged for the individual repetitions. The calculated values are used for a comparison of the mutual content of the detected protein in various samples. This method is suitable primarily in the case when the main part of the background is derived from strong bond of the antibodies on the cellular components and only a small part of the background is caused by the bond to the Petri dishes, or cultivation flasks, or 12-, or 24-, or 48-, 96-well plates, or glasses.
b) In the second case, the signal provided by the secondary antibody conjugated with a fluorochrome in samples with cells, which were not incubated with the primary antibody and concurrently in samples with cells, which were incubated with the primary antibody, is measured first. The signal in samples without cells is further measured. The signal in samples without cells is subsequently subtracted from the signal provided by the secondary antibody conjugated with a fluorochrome in samples with cells. In the case that the samples without cells were processed in several repetitions, it is preferable to use the average of the measured values of the signals for the calculations. Subsequently, the calculated values of the signals in the samples with cells non-incubated with the primary antibody and in the samples with cells incubated with the primary antibody are divided by the value of the corrected signal of DAPI and/or Hoechst and/or the calculated number of cells. In the case of an embodiment, when several repetitions of the samples with cells incubated and non-incubated with the primary antibody are performed, the average signal provided by secondary antibody conjugated with a fluorochrome and also the average corrected signal provided by DAPI and/or Hoechst and/or the average number of cells from these repetitions is preferable calculated first and this average is then used in these and the subsequent calculations. This method is especially suitable in the case of the samples with cells, which were not incubated with the primary antibody. Subsequently, the signal provided by secondary antibody conjugated with fluorochrome is divided by the corrected signal provided by DAPI and/or Hoechst and/or the calculated number of cells. The calculated values for samples with cells without the primary antibody are deducted from the values calculated for samples with cells incubated with the primary antibody. The calculated values are, if it is necessary and they do not arise directly from the previous calculation, averaged for the individual repetitions. The calculated values are used for a comparison of the content of the proteins in various samples. This method is suitable primarily in the case when a significant part of the background is derived from strong bond on a Petri dish, or cultivation flasks, or 12-, or 24-, or 48-, 96-well plates, or glasses.

The object of the present invention is further a method of determining the amount of EdU and/or BrdU and/or CldU and/or IdU incorporated into DNA and the derived replication activity in cells and tissues using the method according to the present invention, wherein the sample of cells or tissues is first incubated with EdU and/or BrdU and/or CldU and/or IdU; subsequently, the step (i) of incubation with the fluorescent substance selected from the group consisted of Hoechst 33258 and/or Hoechst 33342 and/or Hoechst 34580, and the step (ii) of washing with washing aqueous solution of the method according to the present invention are performed; subsequently, an incubation with proteinase K is conducted; and then the fluorescent signal provided by the fluorescent substance from step (i) is measured; subsequently, step (iii) of the method according to the present invention is performed, and the signal provided by the fluorescent substance from step (i) is measured again; then, the fluorescence signal provided by the fluorescent substance after incubation with proteinase K is compared with the fluorescence signal provided by the fluorescent substance contained in the elution solution. Themeasured signal provided by fluorescent substances is related to the signal reflecting the number of cells measured using the described method of determination of the number of cells. In these cases, in the course of the determination of the amount of the incorporated EdU and/or BrdU and/or IdU and/or CldU and subsequently derived replication activity in cells and tissues, the samples of cells or tissues with incorporated EdU and/or BrdU and/or IdU and/or CldU are incubated with Hoechst, the samples are subsequently washed with the washing solution, an incubation with proteinase K is further performed, after incubation with proteinase K the fluorescent signal provided by Hoechst is measured, subsequently incubation in the elution solution is performed and the signal provided by Hoechst is measured again, further, the amount of EdU and/or BrdU and/or IdU and/or CldU incorporated into the DNA is determined so that the first measured fluorescent signal is reduced by the signal measured in the control samples without cells or tissues and then is divided by the second measured signal reduced by the signal measured in the control samples without cells or tissues.

The correction on the background in the determination of replication activity using EdU and/or BrdU and/or IdU and/or CldU is in some embodiments preferably performed after the measurement of the signal coming from Petri dishes, or cultivation flasks, or 12-, or 24-, or 48-, 96-well plates, or glasses without cells. In the case of the performance of several repetitions, the average signal coming from Petri dishes, or cultivation flasks, or 12-, or 24-, or 48-, 96-well plates, or glasses without cells is preferable determined and the average values of these signals is worked with. This signal is subsequently subtracted from the individual signals of the samples with cells and thus the corrected values are acquired. The relative corrected values from the measurements after the activity of proteinase K are indirectly proportional to the content of EdU and/or BrdU and/or IdU and/or CldU and to the replication activity and directly proportional to the number of cells. The relative corrected values of the Hoechst signals after the addition of the elution solution are directly proportional to the number of cells in the individual samples. In the case that it is necessary to perform a precise determination of the cells in the individual samples, the number of cells is simultaneously determined in some samples, for instance, using a microscope and CCD camera and the average Hoechst signal in a single cell is subsequently calculated. The corrected Hoechst signals after the addition of the solution of elution substances provided by the individual samples are then divided by this signal and the number of cells in the individual samples determined. Alternatively, samples of cells containing a known number of cells, e.g. serially diluted samples of cells are used, which are processed according to the protocol described herein, subsequently the calibration curve is calculated, and then the number of cells in the individual samples is determined. If the samples are prepared in several repetitions, the averages from these repetitions are preferably calculated and used in the further calculation.

The corrected signal measured after the incubation of the samples with proteinase K is further divided by the corrected signal measured after the incubation of the samples in the elution solution or the calculated number of cells. The acquired values are then indirectly proportional to the content of EdU and/or IdU and/or CldU and/or BrdU and also indirectly proportional to the replication activity.

Further disclosed herein are kits for determining the amount of DNA in a sample containing cells, such as:
A kit for determining the amount of DNA in a sample containing cells contains at least one fluorescent substance selected from the group consisting of DAPI, Hoechst 33258, Hoechst 33342 and Hoechst 34580; at least one detergent, preferably selected from the group comprising Triton X-100, saponins, Nonidet P-40, Tween 80 and Tween 20 or their aqueous solutions; and at least one elution solution or a substance for the preparation of the elution solution, wherein the elution solution is selected from the group containing aqueous solution of SDS, LiDS and/or sarkosyl or their combination, and wherein the substance for the preparation of the elution solution is SDS, LiDS and/or sarkosyl. Preferably, this kit further contains a substance for fixation of the sample, preferably ethanol- or methanol-stabilized solution of formaldehyde; and/or proteinase, preferably proteinase K; and/or RNase, preferably RNase A; and/or copper (II) sulphate and hydroquinone.

A kit for determining the amount of EdU and/or BrdU and/or CldU and/or IdU incorporated into the DNA and/or for determining the derived replication activity in a sample containing cells contains at least one fluorescent substance selected from the group consisting of Hoechst 33258, Hoechst 33342 and Hoechst 34580; at least one substance selected from the group consisting of EdU, BrdU, CldU and IdU; at least one detergent, preferably selected from the group including Triton X-100, saponins, Nonidet P-40, Tween 80 and Tween 20 or their aqueous solutions; and at least one the elution solution or substance for the preparation of a elution solution, where the elution solution is selected from the group containing aqueous solution SDS, LiDS, sarkosyl or their combination and where the substance for the preparation of the elution solution is SDS, LiDS, sarkosyl; proteinase, preferably proteinase K; further it preferably contains a substance for the fixation of the sample, preferably an ethanol or methanol stabilized solution of formaldehyde; and further preferably contains copper sulphate and hydroquinone.

The use of the method of determining the amount of DNA in a sample according to the present invention is for determining the number of cells and/or the amount of EdU and/or BrdU and/or IdU and/or CldU incorporated into the DNA and/or the replication activity and/or the amount of RNA and/or the amount of proteins and/or the amount of specific DNA sequences and/or the amount of sugars and/or the amount of lipids and/or the amount of fluorescently-labelled DNA and/or the amount of fluorescently-labelled RNA and/or the amount of fluorescently-labelled proteins per cell.

### Brief Description of the Drawings

Figure 1 The analysis of the signal of DAPI, Hoechst 33342 and Hoechst 33258 in various concentrations of SDS. The signal is normalized for the value of the signal in the usage of 10 % (weight/vol.) SDS = 100 %.
Figure 2 The analysis of the signal of DAPI, Hoechst 33342 and Hoechst 33258 in various concentrations of sarkosyl. The signal is normalized for the value of the signal in the usage of 10 % (weight/vol.) sarkosyl = 100 %.
Figure 3 The ratio of the signal of DAPI, Hoechst 33342 and Hoechst 33258 in various concentrations of SDS and sarkosyl.
Figure 4 Simplified schema of the method of determining the amount of DNA in samples. DAPI and/or Hoechst are marked as fluorescent substances. The steps can but do not have to follow closely one after another.
Figure 5 Simplified schema of the method for determining the number of cells in fixed samples. Further steps can be inserted into the method, preferably e.g. permeabilization using detergents, incubation with proteinase K or incubation in a solution of monovalent copper. DAPI and/or Hoechst are marked as fluorescent substances. The steps can but do not have to follow closely one after another. In the step marked as washing, it is preferable to use aqueous, preferably buffered solutions, e.g. a phosphate buffered solution or 1x PBS.
Figure 6 Simplified schema of the method for determining the number of cells in fixed samples incubated in a solution of monovalent copper. Further steps can be inserted into the method, e.g.
permeabilization using detergents or incubation with proteinase K. DAPI and/or Hoechst are marked as fluorescent substances. The steps can but do not have to follow closely one after another. In the step marked as washing, it is preferable to use aqueous, preferably buffered solutions, e.g. a phosphate buffered solution or 1x PBS.

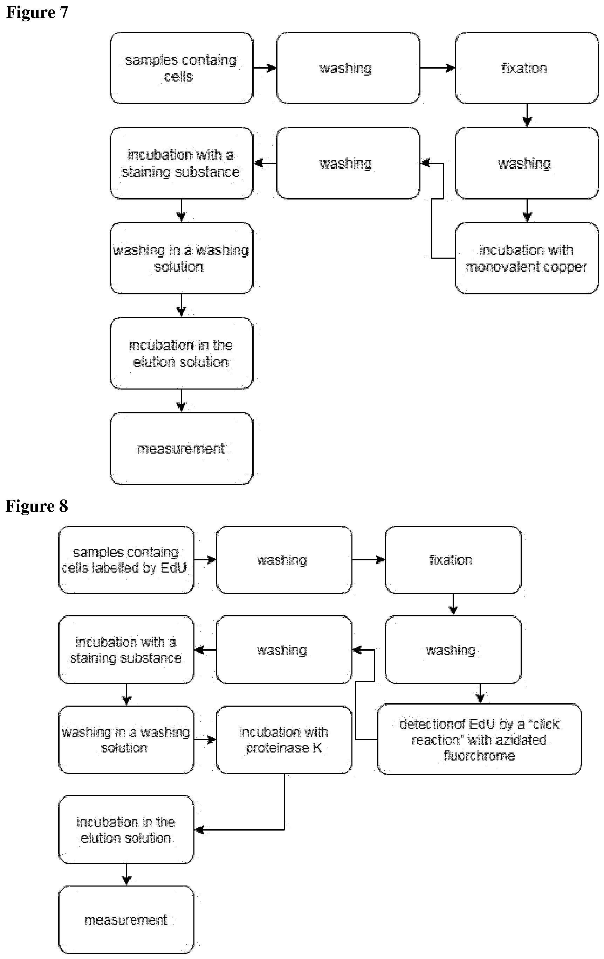

Figure 7 Simplified schema of the method for determining the number of cells in non-fixed samples incubated in a solution of monovalent copper. Further steps can be inserted into the method, preferably e.g. permeabilization using detergents or incubation with proteinase K. DAPI and/or Hoechst are marked as fluorescent substances. The steps can but do not have to follow closely one after another. In the step marked as washing, it is preferable to use aqueous, preferably buffered solutions, e.g. a phosphate buffered solution or 1x PBS.
Figure 8 Simplified schema of the method for determining the number of cells together with the determination of EdU in samples. Further steps can be inserted into the protocol, e.g. permeabilization using detergents. DAPI and/or Hoechst are marked as fluorescence substances. The steps can but do not have to follow closely one after another. In the step marked as washing, it is preferable to use aqueous, preferably buffered solutions, e.g. a phosphate buffered solution or 1x PBS.

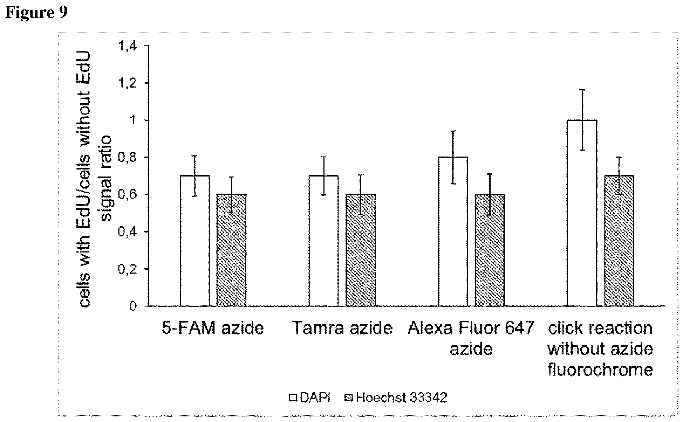

Figure 9 The ratio of the signal of DAPI and Hoechst 33342 in cells incubated in a medium with EdU or without EdU after a reaction with azide fluorochromes. After the addition of the elution substances, this ratio becomes significantly close to the value of 1 in all of the cases.

### List of abbreviations used

- HeLa: cell line of human epithelial cells
- HeLa S3: cell line of human epithelial cells grown in suspension
- DMEM: Dulbecco's modified Eagle's medium
- S-MEM: Spinner modification-Eagle's minimum essential medium for suspension cells
- PBS: phosphate-buffered saline
- EdU: 5-ethynyl-2'-deoxyuridine
- BrdU: 5-bromo-2'-deoxyuridine
- IdU: 5-iodo-2'-deoxyuridine
- CldU: 5-chloro-2'-deoxyuridine

### Examples of the Implementation of the Invention

### Example 1

Determining the amount of DNA in the microtitration plates.

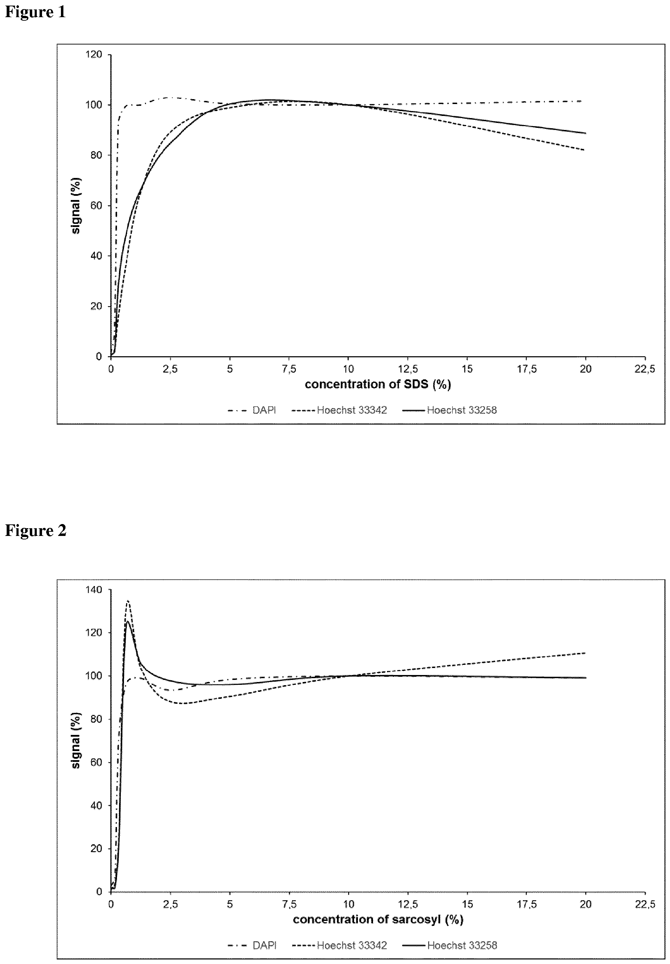

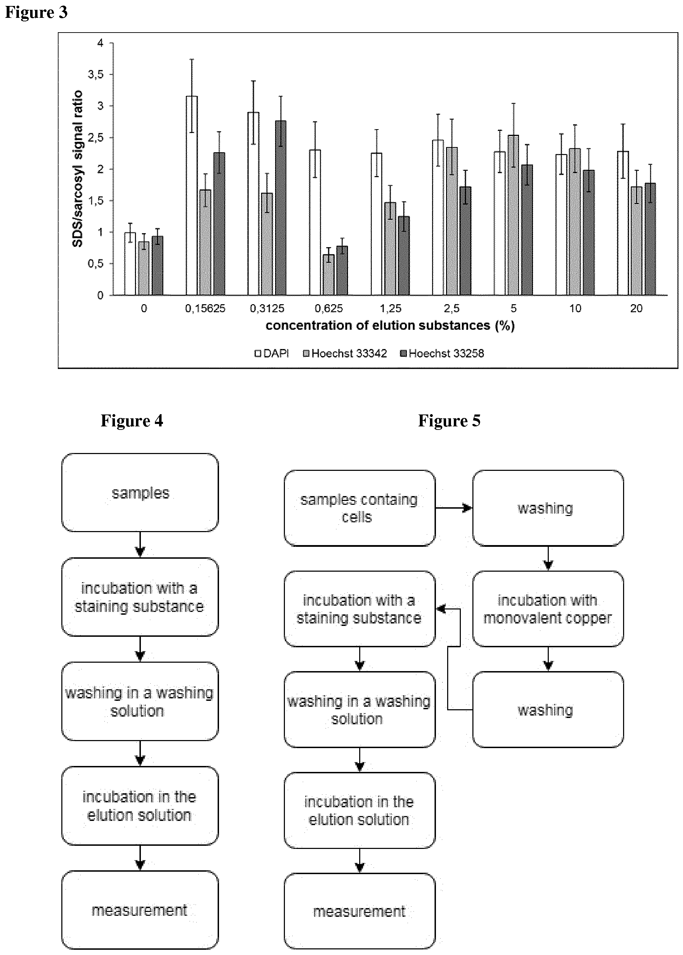

The following method describes the determination of the amount of DNA bound on the surface of the wells of the microtitration plates. If it is not stated otherwise, all of the steps were performed at room temperature, whereas the steps followed one another without any further time delays. For the determination of the background, the entire method was simultaneously performed in wells of microtitration plates without bound DNA.
A) The buffer was removed from the wells of the microtitration plates with bound DNA or without bound DNA and was replaced by a solution of DAPI or Hoechst 33342 or Hoechst 33258 or Hoechst 34580 or a solution containing their combination (alternatively concentrations of fluorescent substances of 0.1; 0.5; 1; 5 or 10 umol.l⁻¹ were used) in 1x PBS or water or in 25 mmol.l⁻¹ Tris-HCl, pH 7.4 and 150 mmol.l⁻¹ NaCl or ethanol or methanol or DMSO or alternatively in 50 % or 70 % of a solution (vol./vol.) of ethanol or methanol in water (approximately 0.2 - 0.4 ml of the solution were added to 1 cm² area, the amount used was derived from the capacity of the well). The samples were alternatively incubated 5, 10 or 30 minutes.
B) The solution of fluorescent substance was removed and replaced by the washing solution: 1x PBS (for the composition, see the section Used solutions and chemicals) without or with the addition alternatively 0.1 %; 0.2 %; 0.4 %; 1 % or 10 % Tween 20 (vol./vol., approximately 0.2 - 0.4 ml of solution to 1 cm² area were added, the amount used was derived from the capacity of the well). The samples were incubated 10 minutes. This step was repeated three more times. In some embodiments, a solution of 25 mmol.l⁻¹ Tris-HCl, pH 7.4 and 150 mmol.l⁻¹ NaCl without or with the addition of Tween 20 or the solution of water without or with the addition of Tween 20 (the used concentrations were the same as in the usage of 1x PBS) was used instead of 1x PBS. In the further embodiments, instead of Tween 20, Triton X-100 (alternatively in the concentration of 0.1 % or 0.2 % (vol./vol.)) or SDS or LiDS or sarkosyl, all alternatively in concentrations of 0.01 %; 0.02 %; 0.03 %; 0.04 % or 0.05 % (weight/vol.) was used.
C) The washing solution was removed and replaced alternatively with 0.1 %; 0.2 %; 1 %; 2 %; 5 %; 10 % or 20 % of the solution of the elution substances (SDS or LiDS or sarkosyl (weight/vol.), approximately 0.2 - 0.4 ml of the solution was added to 1 cm² area, the amount used was derived from the capacity of the well) and the samples were incubated on a laboratory shaker at 300 rpm for the period of alternatively 10, 30 or 60 minutes. The analysis of the signal of DAPI, Hoechst 33342 and Hoechst 33258 in various concentrations of SDS is shown in the Figure 1, in various concentrations of sarkosyl in Figure 2. The ratio of the signal of DAPI, Hoechst 33342 and Hoechst 33258 in various concentrations of SDS and sarkosyl SDS is shown in the Figure 3.
D) The measurement took place directly in the microtitration plate or 20 to 200 µl of the solution was taken and transferred to the wells of the black microtitration plates. The signal was measured using plate reader, preferably at the excitation wave length of 354 - 362 nm in the case of DAPI, at the excitation wave length of 346 - 354 nm in the case of Hoechst 33342 and Hoechst 33258 and at the excitation wave length of 388 - 396 in the case of Hoechst 34580 and also preferably at the emission wave length of 451 - 471 nm for DAPI, Hoechst 33342 and Hoechst 33258 and the emission wave length of 430 - 450 nm with Hoechst 34580. The correction of the background in the case of the signal provided by DAPI and/or Hoechst was performed after measuring the signal coming from the wells of the microtitration plates without bound DNA. In the case of the performance of several repetitions, the average signal in the wells without bound DNA was preferably determined and the average values of these signals were worked with. This signal was subsequently subtracted from the individual signals from the wells with bound DNA. The relative values of these corrected DAPI and/or Hoechst signals from the individual wells then reflected the relative values of the amount of DNA in these wells. A simplified scheme of the method of determination of the amount of DNA is depicted in Figure 4.

### Example 2

Determining the amount of DNA bound in the surface of magnetic particles.

The following method describes the determination of the amount of DNA bound on the surface of magnetic particles. If it is not stated otherwise, all of the steps were performed at room temperature, whereas the steps followed one another without any further time delays. For the determination of the background, the entire method was simultaneously performed with magnetic particles without bound DNA.
A) The magnetic particles with bound DNA or without bound DNA in the test tubes with a volume of 1.5 ml were transferred onto a magnetic separator and were left here for 2 minutes in order to the separation of the magnetic particles with bound DNA or without bound DNA (hereinafter only magnetic particles) from the buffer would take place. The buffer was removed using pipette, the test tubes were transferred onto a normal, non-magnetic stand and 0.5 ml of solution of DAPI or Hoechst 33342 or Hoechst 33258 or Hoechst 34580 was added to the magnetic particles (alternatively concentrations of 0.1; 0.5; 1; 5 or 10 umol.l⁻¹ were used) in 1x PBS or water or in 25 mmol.l⁻¹ Tris-HCl, pH 7.4 and 150 mmol.l⁻¹ NaCl or ethanol or methanol or DMSO or alternatively in 50 % or 70 % of a solution (vol./vol.) of ethanol or methanol in water and the samples were alternatively incubated 5, 10 or 30 minutes on shaker at 300 rpm.
B) The test tubes with magnetic particles were transferred after incubation onto a magnetic separator and were left here for 2 minutes for there in order to the separation of the magnetic particles from the solution would take place. The solution of fluorescent substances was removed using pipette; the test tubes were transferred onto a common, non-magnetic stand and 1 ml of the washing solution was added to the magnetic particles: 1x PBS without or with the addition of alternatively 0.1 %; 0.2 %; 0.4 %; 1 % or 10 % Tween 20 (vol./vol.) and the samples were incubated for 10 minutes This step was repeated three more times. In some embodiments, a solution of water without or with the additive Tween 20 or a solution of 25 mmol.l⁻¹ Tris-HCl, pH 7.4 and 150 mmol.l⁻¹ NaCl without or with the addition of Tween 20 (the concentrations used were the same as in the use of 1x PBS) were used instead of 1x PBS. In other embodiments, Triton X-100 (0.1 % or 0.2 %, (vol./vol.)) or SDS or LiDS or sarkosyl was used instead of Tween 20, all alternatively in concentrations of 0.01 %; 0.02 %; 0.03 %; 0.04 % or 0.05 % (weight/vol.).
C) After incubation, the test tubes with magnetic particles were transferred onto a magnetic separator and were left here for 2 minutes in order to the separation of the magnetic particles from the solution would take place. The washing solution was removed, the test tubes were transferred onto a normal, non-magnetic stand and 0.3 ml of alternatively 0.1 %; 0.2 %; 1 %; 2 %; 5 %; 10 % or 20 % of a solution of the elution substances (SDS or LiDS or sarkosyl (weight/vol.)) was added to the magnetic particles and the samples were incubated on a laboratory shaker at 300 rpm for a period of alternatively 10, 30 or 60 minutes.
D) After incubation, the test tubes with magnetic particles were transferred onto a magnetic separator and were left here for 2 minutes in order to the separation of the magnetic particles from the elution solution would take place. Then, 200 µl of the elution solution was removed and transferred to the wells of the black 96-well microtitration plates. The signal was measured using plate reader, preferably at an excitation wave length of 354 - 362 nm in the case of DAPI, at an excitation wave length of 346 - 354 nm in the case of Hoechst 33342 and Hoechst 33258 and at an excitation wave length of 388 - 396 in the case of Hoechst 34580 and also preferably at an emission wave length of 451 - 471 nm for DAPI, Hoechst 33342 and Hoechst 33258 and an emission wave length of 430 - 450 nm with Hoechst 34580. The correction of the background was conducted by measuring the signal from the samples without bound DNA as in Example 1 Point D.

### Example 3

Determining the number of cells in tissues.

The following method describes the determination of the number of cells in tissues. If it is not stated otherwise, all of the steps were performed at room temperature, whereas the steps followed one after another without any further time delays. For the determination of the background, the entire method was simultaneously performed in the test tube without tissues.
A) The samples of tissues were transferred into the test tube with a volume of 2 ml and were fixed for 30 minutes in a solution of ethanol (1.5 ml of solution of ethanol was added), alternatively 70 % or 100 % (vol./vol.) solution of ethanol was used. Alternatively, the fixation was conducted at -20 °C for a period of 30 minutes or 24 hours or several weeks. In some embodiments, the fixation solution contained DAPI or Hoechst 33342 or Hoechst 33258 or Hoechst 34580 (alternatively, concentrations of 0.1; 0.5; 1; 5 or 10 umol.l⁻¹ were used). In the embodiments, when the solution contained DAPI or Hoechst 33342 or Hoechst 33258 or Hoechst 34580, step D followed.
B) The fixation solution was removed using pipette and was replaced by 1.5 ml of a solution 1x PBS. The solution 1x PBS was removed and replaced by 1.5 ml of a new solution of 1x PBS. This step was repeated one more time.
C) 1x PBS was removed and 0.5 ml of the solution of DAPI or Hoechst 33342 or Hoechst 33258 or Hoechst 34580 was added (alternatively, the concentrations 0.1; 0.5; 1; 5 or 10 umol.l⁻¹ were used) in 1x PBS or in water or in 25 mmol.l⁻¹ Tris-HCl, pH 7.4 and 150 mmol.l⁻¹ NaCl or ethanol or methanol or DMSO or alternatively in 50 % or 70 % of the solution (vol./vol.) of ethanol or methanol in water. The samples were alternatively incubated 5, 10 or 30 minutes on a shaker at 300 rpm.
D) The solution of the fluorescent substance was removed and replaced by the washing solution (1.5 ml): 1x PBS without or with the addition alternatively 0.1 %; 0.2 %; 0.4 %; 1 % or 10 % Tween 20 (vol./vol.) and the samples were incubated 10 minutes. The washing solution was removed and replaced by the new one and the samples were incubated there 10 minutes. This step was repeated two more times. In some embodiments, the solution of water without or with the addition of Tween 20 or 25 mmol.l⁻¹ Tris-HCl, pH 7.4 and 150 mmol.l⁻¹ NaCl without or with the addition of Tween 20 (the used concentrations were the same as in the usage of 1x PBS) was used instead of 1x PBS. In the further embodiments, instead of Tween 20, Triton X-100 (alternatively in the concentration of 0.1 % or 0.2 % (vol./vol.)) or SDS or LiDS or sarkosyl, alternatively in concentrations of 0.01 %; 0.02 %; 0.03 %; 0.04 % or 0.05 % (weight/vol.) was used.
E) The washing solution was removed and replaced with 0,3 ml of alternatively 0.1 %; 0.2 %; 1 %; 2 %; 5 %; 10 % or 20 % of the solution of the elution substances (SDS or LiDS or sarkosyl (weight/vol.)) and the samples were incubated on a laboratory shaker at 300 rpm for the period of alternatively 10, 30 or 60 minutes.
F) 200 µl of the solution was removed from the test tube and transferred to the wells of the black 96-well microtitration plates. The signal was measured using plate reader, preferably at an excitation wave length of 354 - 362 nm in the case of DAPI, at an excitation wave length of 346 - 354 nm in the case of Hoechst 33342 and Hoechst 33258 and at an excitation wave length of 388 - 396 in the case of Hoechst 34580 and also preferably at an emission wave length of 451 - 471 nm for DAPI, Hoechst 33342 and Hoechst 33258 and an emission wave length of 430 - 450 nm with Hoechst 34580. The correction of the background in the case of the signal provided by DAPI and/or Hoechst was performed after measuring the signal coming from the test tube without the tissue sample. This signal was subsequently subtracted from the individual measured signals of samples with tissues. The relative values of these corrected DAPI and/or Hoechst then reflected the relative values of the amount of cells in individual samples. A simplified scheme of the method for determining the number of cells in fixed samples is depicted in Figure 5.

### Example 4

Determining the number of adherent cells after ethanol fixation.

The following method describes determining the number of adherent cells after ethanol fixation. The cultivation of the cells was conducted in Petri dishes, or cultivation flasks, or 12-, or 24-, 48-, or 96-well plates, or on circular glasses with a diameter of 12 mm. If it is not stated otherwise, all of the steps were performed at room temperature, whereas the steps followed one after another without any further time delays. For the determination of the background, the entire method was simultaneously conducted in Petri dishes, or cultivation flasks, or 12-, or 24-, or 48-, 96-well plates, or on glasses without cells. In the case of glasses, after incubation in a medium the glasses were placed in a vessel with a total volume of 2 ml.
A) Human HeLa cells (hereinafter referred to only as samples) were cultivated in a growth medium DMEM with L-glutamine (Gibco), 10 % (vol./vol.), Fetal Bovine Serum (PAA Laboratories), 1 % (vol./vol.) gentamicine and 0.85 g/l NaHCO₃ in an atmosphere with 5% (vol./vol.) CO₂ at 37 °C.
B) The growth medium was subsequently removed and replaced by a buffer (1x PBS, approximately 0.2 - 0.4 ml of the buffer was added on a 1 cm² of the surface, the volume depends on the capacity of the vessel). This step was repeated one more time.
C) The buffer was removed and the samples were fixed for 30 minutes alternatively with 70 % or 100 % (vol./vol.) ethanol solution (approximately 0.2 - 0.4 ml of the buffer was added on a 1 cm² of the surface, the volume depends on the capacity of the vessel). Alternatively, the fixation was performed at -20 °C for a period of 30 minutes or 24 hours or several weeks. In some embodiments, the fixation solution contained DAPI or Hoechst 33342 or Hoechst 33258 or Hoechst 34580 or a solution containing their combination (alternatively, concentrations of 0.1; 0.5; 1; 5 or 10 gmol.l⁻¹ were used). In the embodiments, when the solution contained DAPI or Hoechst 33342 or Hoechst 33258 or Hoechst 34580, step H followed.
D) The fixation solution was removed and replaced by 1x PBS (approximately 0.2 - 0.4 ml of the buffer was added on a 1 cm² of the surface, the volume depends on the capacity of the vessel). This step was repeated two more time.
E) The buffer was removed and replaced with a solution of monovalent copper (for the composition, see the section Solutions and chemicals used, approximately 0.2 - 0.4 ml of the buffer was added on a 1 cm² of the surface, the volume depends on the capacity of the vessel) and the samples were incubated for 20 minutes.
F) The solution of monovalent copper was removed and replaced by 1x PBS (approximately 0.2 - 0.4 ml of the buffer was added on a 1 cm² of the surface, the volume depends on the capacity of the vessel). This step was repeated two more times.
G) The buffer was removed and replaced by a solution of DAPI or Hoechst 33342 or Hoechst 33258 or Hoechst 34580 or with a solution containing their combination (alternatively, concentrations of 0.1; 0.5; 1; 5 or 10 umol.l⁻¹ were used) in 1x PBS or water or 25 mmol.l⁻¹ of Tris-HCl and 150 mmol.l⁻¹ NaCl, pH 7.4 or ethanol or methanol or DMSO or alternatively in 50 % or 70 % of solution (vol./vol.) of ethanol or methanol in water (approximately 0.2 - 0.4 ml of solution for 1 cm² of area were added, the amount was derived from the capacity of the vessel). The samples were alternatively incubated for 5, 10 or 30 minutes.
H) The solution was removed and replaced by the washing solution, which contained 1x PBS without or with alternatively 0.1 %; 0.2 %; 0.4 %; 1 % or 10 % of Tween 20 (vol./vol., approximately 0.2 - 0.4 ml of solution for 1 cm² of area were added, the amount was derived from the capacity of the vessel) and the samples were incubated 10 minutes. This step was repeated three more times.
   In some embodiments, a solution of water without or with Tween 20 (the concentrations used were the same as in the use of 1x PBS) or a solution of 25 mmol.l⁻¹ of Tris-HCl, pH 7.4 and 150 mmol.l⁻¹ NaCl without or with Tween 20 (the concentrations used were the same as in the use of 1x PBS) was used instead of 1x PBS. In other embodiments, Triton X-100 (alternatively 0.1 % or 0.2 %, (vol./vol.)) or SDS or LiDS or sarkosyl; all of them alternatively in concentrations of 0.01 %; 0.02 %; 0.03 %; 0.04 % or 0.05 % (weight/vol.) were used instead of Tween 20.
I) The washing solution was removed and replaced with a solution of proteinase K (for the composition, see the section Used solutions and chemicals, approximately 0.2 - 0.4 ml of solution for 1 cm² of area were added) and the samples were incubated 30 or 60 minutes on a laboratory shaker at 300 rpm at 37 °C. Alternatively, the samples were incubated at a temperature of 56 °C.
J) Alternatively, 10 % or 20 % solution of the elution substances (SDS or LiDS or sarkosyl, (weight/vol.)) was added to the solution of proteinase K. The size of the added volume was alternatively equal to one-ninth, to a quarter of or to the whole volume of the solution of proteinase K. Alternatively, a combination of the elution substances was used. The samples were incubated on a laboratory shaker at 300 rpm alternatively for 10, 30 or 60 minutes.
K) 20 to 200 µl of the solution was transferred to the wells of the black microtitration plates. The signal was measured using plate reader, preferably at the excitation wave length of 354 - 362 nm in the case of DAPI, at the excitation wave length of 346 - 354 nm in the case of Hoechst 33342 and Hoechst 33258 and at the excitation wave length of 388 - 396 in the case of Hoechst 34580 and also preferably at the emission wave length of 451 - 471 nm for DAPI, Hoechst 33342 and Hoechst 33258 and the emission wave length of 430 - 450 nm with Hoechst 34580. The correction of the background in the case of the signal provided by DAPI and/or Hoechst was performed after measuring the signal coming from the Petri dishes or culture flasks or 12-, or 24-, or 48-, 96- microtitration plates, or glasses without cells. In the case of the performance of several repetitions, the average signal in the wells of the Petri dishes or culture flasks or 12-, or 24-, or 48-, 96- microtitration plates, or glasses without cells was preferably determined and the average values of these signals were worked with. This signal was subsequently subtracted from the individual signals from the samples with cells. The relative values of these corrected DAPI and/or Hoechst signals then reflected the relative values of the amount of cells in individual wells.

In the case that it was necessary to carry out a precise determination of the cells in the individual samples, the number of cells in some samples was determined at the same time using a microscope and CCD camera and the average DAPI and/or Hoechst signal in a single cell was subsequently calculated. The corrected signals of DAPI and/or Hoechst provided by the individual samples were then divided by this signal and the number of cells in the individual samples was determined. Alternatively, the samples of the cells containing a known number of cells were used, e.g. serially diluted samples of cells, that were processed according to the protocol described here and the calibration curve was calculated and the number of cells in the individual samples was subsequently determined.

In some embodiments, the incubation step with the solution of monovalent copper and the subsequent washing step were omitted.

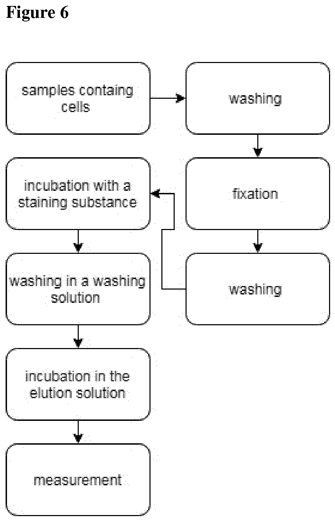

In some embodiments, elution substances were added to the solution of proteinase K or the step with proteinase K was entirely omitted and instead of the step with proteinase K and the subsequent addition of the elution substances, a step was used, in which the incubation was conducted in a solution of the elution substances (SDS, or LiDS or sarkosyl) without the presence of proteinase K. The concentration of the elution substances in this step was alternatively 0.1 %; 0.2 %; 1 %; 2 %; 5 %; 10 % or 20% (weight/vol.) and the samples were incubated on a laboratory shaker at 300 rpm for a period of alternatively 5, 10, 30 or 60 minutes. In some embodiments, 20 to 200 µl of the solution was removed just after incubation with proteinase K and transferred into the wells of black 96-well plates and the solution of the elution substances was only added subsequently. A simplified scheme of the method for determining the number of cells in fixed samples incubated in a monovalent copper solution is depicted in Figure 6.

### Example 5

Determining the number of cells grown in suspension after ethanol fixation.

The following method describes determining the number of cells grown in suspension after ethanol fixation. The cultivation was conducted in Petri dishes, or cultivation flasks, or 12-, or 24-, or 48-, 96-well plates. If it is not stated otherwise, all of the steps were performed at room temperature, whereas the steps followed one after another without any further time delays. For the determination of the background, the entire method was simultaneously conducted in Petri dishes, or cultivation flasks, or 12-, or 24-, or 48-, 96-well plates without cells.
A) Human HeLa S3 cells (hereinafter referred to only as samples) were cultivated in the growth medium S-MEM (Sigma) with 3 % (weight/vol.) of L-glutamine, 5 % (vol./vol.) of Fetal Bovine Serum (PAA Laboratories) and 50 µg/ml of gentamicine in an atmosphere with 5 % (vol./vol.) of CO₂ at 37 °C. The cellular suspension contained approximately 5-8 x 10⁵ cells per 1 millilitre.
B) The second day after the passage, the suspension of the cells was transferred to a 15-millilitre centrifuge test tube (in the case of cultivation in Petri dishes or cultivation flasks) and the samples were centrifuged for 10 minutes at 300g. In the case of cultivation of cells in cultivation plates, the samples were directly centrifuged for 10 minutes at 300g. The supernatant was removed (200 µl of the medium was left in the test tube, all medium was removed from the cultivation plate) and a solution of 1x PBS was added to the samples (10 ml/test tube, 0.2 - 0.4 ml of the buffer was added on a 1 cm² of the surface, the volume depends on the capacity of the vessel). The samples were re-suspended and subsequently centrifuged again for 10 minutes at 300g. The supernatant was removed (200 µl of the medium was left in the test tube, all buffer was removed from the cultivation plate).
C) The subsequent procedure was same as in Example 4 steps C to K whereas during removals of all of the solutions, additions of new solutions and rinsing, the cells were centrifuged for 10 minutes at 300g, the supernatant was subsequently removed (200 µl of the solution was left in the test tube, all the solution was removed from the cultivation plate), the new solution was added to the cells according to the specification in the Example 4 (2 ml/test tube, 0.2 - 0.4 ml of the solution on a 1 cm² of the surface, the volume depends on the capacity of the well), the cells were re-suspended in it and incubated for the periods listed in the individual steps of Example 4.

### Example 6

Determining the number of adherent cells after formaldehyde fixation.

The following method describes determining the number of adherent cells after formaldehyde fixation. The cultivation was conducted in Petri dishes, or cultivation flasks, or 12-, or 24-, or 48-, 96-well plates, or on circular glasses with a diameter of 12 mm. If it is not stated otherwise, all of the steps were performed at room temperature, whereas the steps followed one after another without any further time delays. For the determination of the background, the entire method was simultaneously conducted in Petri dishes, or cultivation flasks, or 12-, or 24-, or 48-, 96-well plates, or on glasses without cells. In the case of glasses, after incubation in a medium the glasses were placed in a vessel with a total volume of 2 ml.

The method was the same as in the Example 4 with the following differences:
Instead of incubation in ethanol the samples were incubated 10 minutes in 2 % (weight/vol.) solution of formaldehyde in 1x PBS (pH 7.4; approximately 0.2 - 0.4 ml of the solution was added on a 1 cm² of the surface, the volume depends on the capacity of the vessel) and were subsequently washed in 1x PBS (3x, approximately 0.2 - 0.4 ml of the solution was added on a 1 cm² of the surface, the volume depends on the capacity of the vessel). In the following step, samples were incubated 10 minutes with 0.1 % Triton X-100 in 1x PBS (approximately 0.2 - 0.4 ml of the solution was added on a 1 cm² of the surface, the volume depends on the capacity of the vessel) and were subsequently washed in 1x PBS (3x, approximately 0.2 - 0.4 ml of the solution was added on a 1 cm² of the surface, the volume depends on the capacity of the vessel).

In some embodiments, the incubation step with Triton X-100 was not performed.

### Example 7

Determining the number of cells grown in a suspension after formaldehyde fixation.

The following method describes determining the number of cells grown in suspension after formaldehyde fixation. The cultivation was conducted in Petri dishes, or cultivation flasks, or 12-, or 24-, or 48-, 96-well plates. If it is not stated otherwise, all of the steps were performed at room temperature, whereas the steps followed one after another without any further time delays. For the determination of the background, the entire method was simultaneously conducted in Petri dishes, or cultivation flasks, or 12-, or 24-, or 48-, 96-well plates without cells.

The method was the same as in Example 5 with the following changes:
Instead of incubation in ethanol the samples were incubated 10 minutes in 2 % (weight/vol.) solution of formaldehyde in 1x PBS (pH 7.4; 10 ml/test tube, 0.2 - 0.4 ml of the solution on a 1 cm² of the surface, the used volume depends on the capacity of the well of the cultivation plate) and were subsequently washed in 1x PBS (3x, 10 ml/test tube, 0.2 - 0.4 ml of the solution on a 1 cm² of the surface, the used volume depends on the capacity of the well of the cultivation plate). In the following step, samples were incubated 10 minutes with 0.1 % Triton X-100 in 1x PBS (10 ml/test tube, 0.2 - 0.4 ml of the solution on a 1 cm² of the surface, the used volume depends on the capacity of the well of the cultivation plate) and were subsequently washed in 1x PBS (3x, 10 ml/test tube, 0.2 - 0.4 ml of the solution on a 1 cm² of the surface, the used volume depends on the capacity of the well of the cultivation plate).

In some embodiments the incubation step with Triton X-100 was not performed.

### Example 8

Determining the number of cells after methanol fixation.

The following method describes determining the number of cells after methanol fixation. The cultivation was conducted in Petri dishes, or cultivation flasks, or 12-, or 24-, or 48-, 96-well plates, and in the case of adherent cells also on circular glasses with a diameter of 12 mm. If it is not stated otherwise, all of the steps were performed at room temperature, whereas the steps followed one after another without any further time delays. For the determination of the background, the entire method was simultaneously conducted in Petri dishes, or cultivation flasks, or 12-, or 24-, or 48-, 96-well plates, or in the case of adherent cells, on glasses without cells. In the case of glasses, after incubation in a medium the glasses were placed in a vessel with a total volume of 2 ml.

The method was the same as in Examples 4 and 5 with the following changes:
Instead of the fixation with ethanol, methanol was used.

### Example 9

Determining the number of unfixed cells incubated with a solution of monovalent copper.

The following method describes determining the number of unfixed cells incubated with a solution of monovalent copper. The cultivation was conducted in Petri dishes, or cultivation flasks, or 12-, or 24-, or 48-, 96-well plates, and in the case of adherent cells also on circular glasses with a diameter of 12 mm. If it is not stated otherwise, all of the steps were performed at room temperature, whereas the steps followed one after another without any further time delays. For the determination of the background, the entire method was simultaneously conducted in Petri dishes, or cultivation flasks, or 12-, or 24-, or 48-, 96-well plates, or on glasses without cells. In the case of glasses, after incubation in a medium the glasses were placed in a vessel with a total volume of 2 ml.

The method was the same as in Examples 4 and 5 with the following changes:
Fixation using ethanol and also the subsequent washing of the samples in 1x PBS was omitted. The simplified scheme of the method of determining the number of cells in unfixed samples incubated in a solution of monovalent copper is depicted in Figure 7.

### Example 10

Determining the number of unfixed adherent cells.

The following method describes determining the number of unfixed adherent cells. The cells were cultivated in Petri dishes, or cultivation flasks, or 12-, or 24-, or 48-, 96-well plates, or on circular glasses with a diameter of 12 mm. If it is not stated otherwise, all of the steps were performed at room temperature, whereas the steps followed one after another without any further time delays. For the determination of the background, the entire method was simultaneously conducted in Petri dishes, or cultivation flasks, or 12-, or 24-, or 48-, 96-well plates, or on glasses without cells. In the case of glasses, after incubation in a medium the glasses were placed in a vessel with a total volume of 2 ml.
A) Human HeLa cells (hereinafter referred to only as samples) were cultivated in a growth medium DMEM with L-glutamine (Gibco), 10 % (vol./vol.) Fetal Bovine Serum (PAA Laboratories), 1 % (vol./vol.) gentamicine and 0.85 g/l NaHCO₃ in an atmosphere with 5% (vol./vol.) CO₂ at 37 °C.
B) The growth medium was removed and replaced with a solution of Hoechst 33342 or Hoechst 33258 or Hoechst 34580 in 1x PBS (alternatively 0.1; 0.5; 1, 5 or 10 umol.l⁻¹ concentrations of Hoechst were used, approximately 0.2 - 0.4 ml of the solution was added on a 1 cm² of the surface, the amount used was derived from the capacity of the well) and the samples were incubated alternatively for 5 or 10 or 30 minutes.
C) The Hoechst solution was removed and replaced by a washing solution buffer 1x PBS (approximately 0.2 - 0.4 ml of the buffer was added on a 1 cm² of the surface, the amount used was derived from the capacity of the well) and the samples were incubated for 5 minutes. This step was repeated three more times.
D) The washing solution was removed and replaced alternatively with 0.1 %; 0.2 %; 1 %; 2 %; 5 %; 10 % or 20 % of the solution of the elution substances (SDS or LiDS or sarkosyl (weight/vol.), approximately 0.2 - 0.4 ml of the solution was added to 1 cm² area, the amount used was derived from the capacity of the well) and the samples were incubated on a laboratory shaker at 300 rpm for the period of alternatively 10, 30 or 60 minutes.
E) 200 µl of the solution was removed and transferred to the wells of the black microtitration plate. The signal was measured using plate reader, preferably at the excitation wave length of 354 - 362 nm in the case of DAPI, at the excitation wave length of 346 - 354 nm in the case of Hoechst 33342 and Hoechst 33258 and at the excitation wave length of 388 - 396 in the case of Hoechst 34580 and also preferably at the emission wave length of 451 - 471 nm for DAPI, Hoechst 33342 and Hoechst 33258 and the emission wave length of 430 - 450 nm with Hoechst 34580. The correction of the background was performed by measuring of the signal from vessels without cells and it was performed as in the Example 4.

### Example 11

Determining the number of unfixed cells grown in suspension.

The following method describes determining the number of unfixed cells grown in suspension. The cells were cultivated in Petri dishes, or cultivation flasks, or 12-, or 24-, or 48-, 96-well plates. If it is not stated otherwise, all of the steps were performed at room temperature, whereas the steps followed one after another without any further time delays. For the determination of the background, the entire method was simultaneously conducted in Petri dishes, or cultivation flasks, or 12-, or 24-, or 48-, 96-well plates without cells.
A) Human HeLa S3 cells (hereinafter referred to only as samples) were cultivated in the growth medium S-MEM (Sigma) with 3 % (weight/vol.) of L-glutamine, 5 % (vol./vol.) of Fetal Bovine Serum (PAA Laboratories) and 50 µg/ml of gentamicine in an atmosphere with 5 % (vol./vol.) of CO₂ at 37 °C. The cellular suspension contained approximately 5-8 x 10⁵ cells per 1 millilitre.
B) The second day after the passage, the suspension of the cells was transferred to a 15-millilitre centrifuge test tube (in the case of cultivation in Petri dishes or cultivation flasks) and the samples were centrifuged for 10 minutes at 300g. In the case of cultivation of cells in cultivation plates, the samples were directly centrifuged for 10 minutes at 300g. The supernatant was removed (200 µl of the medium was left in the test tube, all medium was removed from the cultivation plate).

The subsequent procedure was same as in Example 10 whereas during removals of all of the solutions, additions of new solutions and rinsing, the cells were centrifuged for 10 minutes at 300g, the supernatant was subsequently removed (200 µl of the solution was left in the test tube, all the solution was removed from the cultivation plate), the new solution was added to the cells according to the specification in the Example 10 (2 ml/test tube, 0.2 - 0.4 ml of the solution on a 1 cm² of the surface, the volume depends on the capacity of the well of the cultivation plate), the cells were re-suspended in it and incubated for the periods listed in the individual steps of Example 10.

### Example 12

Determining the relative replication activity and the replication activity related to a cell.

The following method describes the determination of the relative replication activity and the replication activity related to a cell using EdU in Petri dishes, or cultivation flasks, or 12-, or 24-, or 48-, 96-well plates, or on circular glasses with a diameter of 12 mm. If it is not stated otherwise, all of the steps were performed at room temperature, whereas the steps followed one after another without any further time delays. For determining the background caused by the bond of DAPI and/or Hoechst and/or azide fluorochromes on the cultivation and incubation surfaces, the whole method was simultaneously conducted in Petri dishes, or cultivation flasks, or 12-, or 24-, or 48-, 96-well plates, or with glasses without cells. In the case of glasses, after incubation in a medium the glasses were placed in a vessel with a total volume of 2 ml.
A) Human HeLa cells (hereinafter referred to only as samples) were cultivated in a growth medium DMEM with L-glutamine (Gibco), 10 % (vol./vol.), Fetal Bovine Serum (PAA Laboratories), 1 % (vol./vol.) gentamicine and 0.85 g/l NaHCO₃ in an atmosphere with 5% (vol./vol.) CO₂ at 37 °C.
B) On the second day, 10 umol.l⁻¹ EdU was added and cells were incubated for 30 minutes in an atmosphere with 5% (vol./vol.) CO₂ at 37 °C. In some embodiments, the addition of EdU to the samples was omitted. In another embodiments, EdU was added also to the samples without cells.
C) The growth medium was subsequently removed and replaced by a buffer (1x PBS, approximately 0.2 - 0.4 ml of the buffer was added on a 1 cm² of the surface, the volume depends on the capacity of the vessel). This step was repeated one more time.
D) The buffer was removed and the samples were alternatively fixed with 70 % (vol./vol.) ethanol solution in 1x PBS, (pH 7,4, or 100 % ethanol(approximately 0.2 - 0.4 ml of the fixation solution was added on a 1 cm² of the surface, the volume depends on the capacity of the vessel)for 30 minutes. Alternatively, the fixation was performed at -20 °C for a period of 30 minutes or 24 hours or several weeks.
E) Fixation solution was removed and replaced by 1x PBS (approximately 0.2 - 0.4 ml of the buffer was added on a 1 cm² of the surface, the volume depends on the capacity of the vessel). This step was repeated two more times.
F) The buffer was removed and replaced with a solution of monovalent copper with 10 10 gmol.l⁻¹ fluorochrome azide (5-FAM; for the composition, see the section Solutions and chemicals used, approximately 0.2 - 0.4 ml of the solution was added on a 1 cm² of the surface, the used volume depends on the capacity of the vessel, examples of another fluorochrome azides are described in the Disclosure of the Invention part) and the samples were incubated for 20 minutes.
G) The solution of monovalent copper was removed and replaced by 1x PBS (approximately 0.2 - 0.4 ml of the buffer was added on a 1 cm² of the surface, the volume depends on the capacity of the vessel). This step was repeated two more times.
H) The buffer was removed and was replaced by a solution of DAPI or Hoechst 33342 or Hoechst 33258 or Hoechst 34580 (alternatively concentrations of 0.1; 0.5; 1; 5 or 10 umol.l⁻¹ were used) in 1x PBS or in 25 mmol.l⁻¹ Tris-HCl, pH 7.4 and 150 mmol.l⁻¹ NaCl (approximately 0.2 - 0.4 ml of the solution were added to 1 cm² area, the amount used was derived from the capacity of the vessel) and the samples were alternatively incubated 5, 10 or 30 minutes.
I) The solution was removed and replaced by the washing solution: 1x PBS without or with the addition alternatively 0.1 %; 0.2 %; 0.4 %; 1 % or 10 % Tween 20 (vol./vol., approximately 0.2 - 0.4 ml of solution to 1 cm² area were added, the amount used was derived from the capacity of the vessel) and the samples were incubated 10 minutes. This step was repeated three more times. In some embodiments, a solution of water without or with the addition of Tween 20 or the solution of 25 mmol.l⁻¹ Tris-HCl, pH 7.4 and 150 mmol.l⁻¹ NaCl without or with the addition of Tween 20 (the used concentrations were the same as in the usage of 1x PBS) was used instead of 1x PBS. In the further embodiments, instead of Tween 20, Triton X-100 (alternatively in the concentration of 0.1 % or 0.2 % (vol./vol.)) or SDS or LiDS or sarkosyl, all of them alternatively in concentrations of 0.01 %; 0.02 %; 0.03 %; 0.04 % or 0.05 % (weight/vol.), were used.
J) The washing solution was removed and replaced with a solution of proteinase K (approximately 0.2 - 0.4 ml of solution for 1 cm² of area were added, the amount used was derived from the capacity of the vessel) and the samples were incubated 30 or 60 minutes on a laboratory shaker at 300 rpm at 37 °C. Alternatively, the samples were incubated at a temperature of 56 °C.
K) Alternatively, 10 % or 20 % aqueous solution of the elution substances (SDS or LiDS or sarkosyl, (weight/vol.) was added to the solution of proteinase K; the size of the added volume was alternatively equal to one-ninth, to a quarter or to the whole volume of the solution of proteinase K). The samples were incubated on a laboratory shaker at 300 rpm alternatively for 10, 30 or 60 minutes.
L) 120 µl of the solution was removed and transferred to the wells of the black 96-well microtitration plate. The signal was measured using plate reader, preferably at an excitation wave length of 354 - 362 nm in the case of DAPI, at an excitation wave length of 346 - 354 nm in the case of Hoechst 33342 and Hoechst 33258 and at an excitation wave length of 388 - 396 in the case of Hoechst 34580 and also preferably at an emission wave length of 451 - 471 nm for DAPI, Hoechst 33342 and Hoechst 33258 and an emission wave length of 430 - 450 nm with Hoechst 34580. The correction of the background in the case of the signal provided by DAPI and/or Hoechst was performed after measuring the signal coming from the Petri dishes, or culture flasks, or 12-, or 24-, or 48-, 96-well plates, or glasses without cells. In the case of the performance of several repetitions, the average signal in the wells of the Petri dishes or culture flasks or 12-, or 24-, or 48-, 96- microtitration plates, or glasses without cells was preferably determined and the average values of these signals were worked with. This signal was subsequently subtracted from the individual signals from the samples with cells. The relative values of these corrected DAPI and/or Hoechst signals then reflected the relative values of the amount of cells in individual wells.

In the case that it was necessary to perform a precise determination of the cells in the individual samples, the number of cells in some samples was determined at the same time using a microscope and CCD camera and the average DAPI and/or Hoechst signal in a single cell was subsequently calculated. The corrected DAPI and/or Hoechst signals provided by the individual samples with cells were then divided by this signal and the number of cells in the individual samples was determined. Alternatively, serially diluted samples with cells, which were processed according to the protocol described here, were used; the calibration curve was calculated and the number of cells in the individual samples was subsequently determined.

The signal provided by the azide fluorochrome (5-FAM) reacting with EdU was also measured using plate reader at the excitation wave length of 490 - 498 nm and emission wave length of 510 - 530 nm. Subsequently, the correction of the background of this signal was or was not conducted. The determination of replication activity and the correction were conducted in several ways:
- In the case that the correction of the background of the signal provided by azide fluorochrome (5-FAM) reacting with EdU was not conducted, the signal provided by azide fluorochrome (5-FAM) reacting with EdU was divided by the corrected signal provided by DAPI and/or Hoechst and/or the calculated number of cells. In some embodiments, in the case of the performance of several repetitions, the average signal provided by azide fluorochrome (5-FAM) reacting with EdU and the average corrected signal provided by DAPI and/or Hoechst and/or the average number of cells in these samples were determined and only subsequently the average signal provided by azide fluorochrome (5-FAM) reacting with EdU was divided by the average corrected signal provided by DAPI and/or Hoechst and/or the average number of cells. The calculated value was then used for a comparison of the mutual replication activity in various samples. This method is suitable primarily in the case when the used azide fluorochrome reacting with EdU does not bind strongly to the cellular structures, and/or to the Petri dishes, or to the cultivation flasks, or to the 12-, or 24-, or 48-, 96-well plates, or on the glasses, and/or they can be easily removed by washing.
- In the case that it was necessary to carry out a correction on the background, it was proceeded in two different ways.

In the first case, the signal provided by azide fluorochrome (5-FAM) in the samples with cells, which were cultivated with EdU, and simultaneously in the samples with cells, which were not cultivated with EdU, was first measured. Subsequently, the signal in the samples with cells cultivated without EdU and in the samples with cells cultivated with EdU was divided by the value of the corrected signal of DAPI and/or Hoechst and/or the calculated number of cells. In the case when several repetitions of the samples with cells cultivated and not cultivated with EdU was carried out, preferably, the average signal provided by azide fluorochrome (5-FAM) reacting with EdU and also the average corrected the signal provided by DAPI and/or Hoechst and/or the average number of cells from these repetitions was calculated first and this was then used in these and the subsequent calculations. This method was especially suitable in the case of cells, which were cultivated in a medium without EdU. Subsequently, the signal provided by azide fluorochrome (5-FAM) reacting with EdU was divided by the corrected signal provided by DAPI and/or Hoechst and/or the calculated number of cells. The calculated values for samples with cells cultivated without EdU were subtracted from the values calculated for the samples with cells cultivated with EdU. The calculated values were, if it was necessary and did not arise directly from the previous calculation, averaged for the individual repetitions. The calculated values were used for a comparison of the mutual replication activity in various samples. This method is suitable primarily in the case when the main part of the background is derived from the strong bond of the azide fluorochromes to the cellular components and only a small part of the background is caused by their bond to the Petri dishes, or cultivation flasks, or 12-, or 24-, or 48-, 96-well plates, or glasses.

In the second case, the signal provided by azide fluorochrome (5-FAM) reacting with EdU in the samples with cells, which were cultivated with EdU and simultaneously in the samples with cells, which were not cultivated with EdU, was first measured. Furthermore, the signal in samples without cells was measured. The signal in samples without cells was subsequently subtracted from the signal provided by azide fluorochrome (5-FAM) reacting with EdU in samples with cells. In the case that the samples without cells were processed in several repetitions, the average of the measured values of the signals was preferably used for the further calculations. Subsequently, the calculated values of the signals in the samples with cells cultivated without EdU and in the samples with cells cultivated with EdU were divided by the value of the corrected signal of DAPI and/or Hoechst and/or the calculated number of cells. In the embodiment, when several repetitions were performed on samples with cells cultivated and uncultivated with EdU, the average signal of azide fluorochrome (5-FAM) reacting with EdU and also the average corrected signal provided by DAPI and/or Hoechst and/or the average number of cells from these repetitions was preferably calculated first and this was used in these and the subsequent calculations. This method was especially suitable in the case of the samples with cells, which were cultivated without EdU. Subsequently, the signal provided by azide fluorochrome (5-FAM) reacting with EdU was divided by the corrected signal provided by DAPI and/or Hoechst and/or the calculated number of cells. The calculated values for samples with cells cultivated without EdU were subtracted from the values calculated for the samples with cells cultivated with EdU. The calculated values were, if it was necessary and did not arise directly from the previous calculation, averaged for the individual repetitions. The calculated values were used for a comparison of the mutual replication activity in various samples. This method is suitable primarily in the case when the main part of the background is derived from strong bond of azide fluorochromes to the Petri dishes, or cultivation flask, or 12-, or 24-, or 48-, 96-well plates, or glasses.

In some embodiments, elution substances were added to the solution of proteinase K. The concentrations of the elution substances in this step were alternatively 0.1 %, 0.2 %;; 1 %; 2 %; 5 %; 10 % or 20 % (weight/vol.) and the samples were incubated on a laboratory shaker at 300 rpm for a period of alternatively 10, 30 or 60 minutes. In other embodiments, 120 µl of the samples was transferred immediately after incubation with proteinase K into the wells of black 96-well plates and only then was the solution of the elution substances added to the individual samples. In some embodiments, methanol was used instead of ethanol or a fixation with formaldehyde and permeabilization with Triton X-100 was used following Example 6. A simplified scheme of the method of determination of the number of cells together with the determination of the amount of EdU in samples is depicted in Figure 8. The ratio of DAPI and Hoechst 33342 signal in cells incubated in EdU-containing medium and in EdU-non-containing medium after the reaction with azide fluorochromes is depicted in Figure 9.

### Example 13

Determining the content of EdU and/or BrdU and/or CldU and/or IdU in the DNA of cells and the derived replication activity of these cells.

The following method describes the determination of the content of EdU and/or BrdU and/or CldU and/or IdU in the DNA of adherent cells and the derived replication activity of these cells in Petri dishes, or cultivation flasks, or 12-, or 24-, or 48-, 96-well plates, or on circular glasses with a diameter of 12 mm. If it is not stated otherwise, all of the steps were performed at room temperature, whereas the steps followed one after another without any further time delays. For determining the background caused by the bond of Hoechst on the cultivation and incubation surfaces, the whole method was simultaneously conducted in Petri dishes, or cultivation flasks, or 12-, or 24-, or 48-, 96-well plates, or with glasses without cells. In the case of glasses, after incubation in a medium the glasses were placed in a vessel with a total volume of 2 ml.
A) Human HeLa cells (hereinafter referred to only as samples) were cultivated in a growth medium DMEM with L-glutamine (Gibco), 10 % (vol./vol.), Fetal Bovine Serum (PAA Laboratories), 1 % (vol./vol.) gentamicine and 0.85 g/l NaHCO₃ in an atmosphere with 5% (vol./vol.) CO₂ at 37 °C.
B) On the second day, 10 umol.l⁻¹ EdU and/or IdU and/or CldU and/or BrdU was added to the medium and cells were incubated alternatively for 8 or 20 hours in an atmosphere with 5% (vol./vol.) CO₂ at 37 °C. In some embodiments, neither EdU nor IdU nor CldU nor BrdU was added into some samples. In other embodiments, EdU and/or IdU and/or CldU and/or BrdU was added also into the samples, where there were no cells.
C) The growth medium was removed and replaced by a buffer (1x PBS, approximately 0.2 - 0.4 ml of the solution was added on a 1 cm² of the surface, the used volume depends on the capacity of the vessel). This step was repeated one more time.
D) The buffer was removed and the samples were alternatively fixed with 70 % (vol./vol.) ethanol solution in 1x PBS, pH 7,4 or 100 % ethanol (approximately 0.2 - 0.4 ml of the fixation solution was added on a 1 cm² of the surface, the volume depends on the capacity of the vessel) for 30 minutes. Alternatively, the fixation was performed at -20 °C for a period of 30 minutes or 24 hours or several weeks.
E) The fixation solution was removed and replaced by 1x PBS (approximately 0.2 - 0.4 ml of the buffer was added on a 1 cm² of the surface, the used volume depends on the capacity of the vessel). This step was repeated two more times.
F) The buffer was removed and replaced with a solution of monovalent copper (approximately 0.2 - 0.4 ml of the solution was added on a 1 cm² of the surface, the used volume depends on the capacity of the vessel) and the samples were incubated for 20 minutes.
G) The solution of monovalent copper was removed and replaced by 1x PBS (approximately 0.2 - 0.4 ml of the solution was added on a 1 cm² of the surface, the used volume depends on the capacity of the vessel). This step was repeated two more times.
H) The buffer was removed and was replaced by a solution of Hoechst 33342 or Hoechst 33258 or Hoechst 34580 (alternatively concentrations of 0.1; 0.5; 1; 5 or 10 umol.l⁻¹ were used) in 1x PBS or in 25 mmol.l⁻¹ Tris-HCl, pH 7.4 and 150 mmol.l⁻¹ NaCl (approximately 0.2 - 0.4 ml of the solution were added to 1 cm² area, the amount used was derived from the capacity of the vessel) and the samples were alternatively incubated 5, 10 or 30 minutes.
I) The solution was removed and replaced by the washing solution: 1x PBS without or with the addition alternatively 0.1 %; 0.2 %; 0.4 %; 1 % or 10 % Tween 20 (vol./vol., approximately 0.2 - 0.4 ml of solution to 1 cm² area were added, the amount used was derived from the capacity of the vessel) and the samples were incubated 10 minutes. This step was repeated three more times. In some embodiments, a solution of water without or with the addition of Tween 20 or the solution of 25 mmol.l⁻¹ Tris-HCl, pH 7.4 and 150 mmol.l⁻¹ NaCl without or with the addition of Tween 20 (the used concentrations were the same as in the usage of 1x PBS) was used instead of 1x PBS. In the further embodiments, , Triton X-100 (alternatively in the concentration of 0.1 % or 0.2 % (vol./vol.)) or SDS or LiDS or sarkosyl, all alternatively in concentrations of 0.01 %; 0.02 %; 0.03 %; 0.04 % or 0.05 % (weight/vol.) was used instead of Tween 20.
J) The washing solution was removed and replaced with a solution of proteinase K (approximately 0.2 - 0.4 ml of solution for 1 cm² of area were added, the amount used was derived from the capacity of the vessel) and the samples were incubated 30 or 60 minutes on a laboratory shaker at 300 rpm at 37 °C. Alternatively, the samples were incubated at a temperature of 56 °C.
K) 120 µl of the solution was removed and transferred to the wells of the black 96-well plate. The signal was measured using plate reader, preferably at an excitation wave length of 346 - 354 nm in the case of Hoechst 33342 and Hoechst 33258, and at an excitation wave length of 388 - 396 in the case of Hoechst 34580, and, also preferably, at an emission wave length of 451 - 471 nm for Hoechst 33342 and Hoechst 33258, and an emission wave length of 430 - 450 nm with Hoechst 34580.
L) 10 % or 20 % of a solution of the elution substances (SDS or LiDS or sarkosyl (weight/vol.) was alternatively added to the solution, the size of the added volume was alternatively equal to one-ninth, to a quarter or to the whole volume of the solution of proteinase K). The samples were incubated on a laboratory shaker at 300 rpm for a period of alternatively 10, 30 or 60 minutes.
M) The signal was measured using plate reader, preferably at an excitation wave length of 346 - 354 nm in the case of Hoechst 33342 and Hoechst 33258, and at an excitation wave length of 388 - 396 in the case of Hoechst 34580, and, also preferably, at an emission wave length of 451 - 471 nm for Hoechst 33342 and Hoechst 33258, and an emission wave length of 430 - 450 nm with Hoechst 34580.

For determining the content of EdU and/or IdU and/or CldU and/or BrdU and of the derived replication activity, the values measured in steps K and M were used. The correction of the background in these values was performed after measuring the signal coming from the Petri dishes, or culture flasks, or 12-, or 24-, or 48-, 96-well plates, or glasses without cells. In the case of the performance of several repetitions, the average signal in the wells of the Petri dishes or culture flasks or 12-, or 24-, or 48-, 96- well plates, or glasses without cells was preferably determined and the average values of these signals were worked with. This signal was subsequently subtracted from the individual signals of the samples with cells and the corrected values were thus acquired. Whereas the relative corrected values from step K were indirectly proportional to the content of EdU and/or IdU and/or CldU and/or BrdU and also indirectly proportional to the replication activity, the corrected values of the Hoechst signals from step M were directly proportional to the number of cells in the individual samples. In the case that it was necessary to carry out a precise determination of the cells in the individual samples, the number of cells in some samples was determined at the same time using a microscope and CCD camera and the average Hoechst signal in a single cell was subsequently calculated. The corrected signals of Hoechst from the step M provided by the individual samples were then divided by this signal and the number of cells in the individual samples was determined. Alternatively, the samples of serially diluted cells which were processed according to the described approach, the calibration curve was calculated and subsequently the number of cells in the individual samples was determined. If the samples were prepared in several repetitions, the averages from these repetitions were preferably calculated and used in the further calculation.

Further, the corrected signal from step K was divided by the corrected signal from step M or the calculated number of cells. The acquired values were then indirectly proportional to the content of EdU and/or IdU and/or CldU and/or BrdU and also indirectly proportional to the replication activity.

In some embodiments, methanol was used instead of ethanol or a fixation with formaldehyde and permeabilization with Triton X-100 was used following Example 6.

### Example 14

Determining the content of the proteins in the cells using an antibody reacting with this protein.

The following method describes the determination of the content of the proteins in the cells cultivated in Petri dishes, or cultivation flasks, or 12-, or 24-, or 48-, 96-well plates, or on circular glasses with a diameter of 12 mm using an antibody reacting with this protein. If it is not stated otherwise, all of the steps were performed at room temperature, whereas the steps followed one after another without any further time delays. For determining the background caused by the bond of DAPI and/or Hoechst and/or antibodies reacting with protein and/or antibodies conjugated with fluorochromes on the cultivation and incubation surfaces, the whole method was simultaneously conducted in Petri dishes, or cultivation flasks, or 12-, or 24-, or 48-, 96-well plates, or with glasses without cells. In the case of glasses, after incubation in a medium the glasses were placed in a vessel with a total volume of 2 ml.
A) Human HeLa cells (hereinafter referred to only as samples) were cultivated in a growth medium DMEM with L-glutamine (Gibco), 10 % (vol./vol.) Fetal Bovine Serum (PAA Laboratories), 1 % (vol./vol.) gentamicine and 0.85 g/l NaHCO₃ in an atmosphere with 5% (vol./vol.) CO₂ at 37 °C.
B) The growth medium was removed and replaced by a buffer (1x PBS, approximately 0.2 - 0.4 ml of the solution was added on a 1 cm² of the surface, the used volume depends on the capacity of the vessel). This step was repeated one more time.
C) The buffer was removed and the samples were fixed for 30 minutes alternatively with 70 % (vol./vol.) ethanol solution in 1x PBS, pH 7,4 or with 100 % ethanol (approximately 0.2 - 0.4 ml of the fixation solution was added on a 1 cm² of the surface, the used volume depends on the capacity of the vessel). Alternatively, the fixation was performed at -20 °C for a period of 30 minutes or 24 hours or several weeks.
D) The fixation solution was removed and replaced by 1x PBS (approximately 0.2 - 0.4 ml of the buffer was added on a 1 cm² of the surface, the used volume depends on the capacity of the vessel). This step was repeated two more times.
E) The buffer was removed and replaced with a solution of monovalent copper (approximately 0.2 - 0.4 ml of the solution was added on a 1 cm² of the surface, the used volume depends on the capacity of the vessel) and the samples were incubated for 20 minutes. In some embodiments, this step and the following step were omitted.
F) The solution of monovalent copper was removed and replaced by 1x PBS (approximately 0.2 - 0.4 ml of the solution was added on a 1 cm² of the surface, the used volume depends on the capacity of the vessel). This step was repeated two more times.
G) The buffer was removed and replaced with a solution of 1x PBS and primary antibody reacting with the detected protein. The optimal concentration of the antibody was determined by serial dilution. Approximately 0.2 - 0.4 ml of the buffer with the antibody was added on a 1 cm² of the surface, the used volume depends on the capacity of the vessel. In some embodiments, the solution of 1x PBS did not contain the antibody.
H) The solution of 1x PBS and antibody was removed and replaced by 1x PBS (approximately 0.2 - 0.4 ml of the solution was added on a 1 cm² of the surface, the used volume depends on the capacity of the vessel). This step was repeated two more times.
I) The buffer was removed and replaced with a solution of a secondary antibody conjugated with the fluorochrome Alexa Fluor 488 whereas this antibody reacted with the antibody from the step G. The concentration of the secondary antibody was 10 µg/ml. The secondary antibody was diluted in 1x PBS or 25 mmol.l⁻¹ Tris-HCl, pH 7.4 and 150 mmol.l⁻¹ NaCl and the solution also contained DAPI and/or Hoechst 33342 and/or Hoechst 33258 and/or Hoechst 34580 (alternatively, 0.1; 0.5; 1; 5 or 10 µmol.l⁻¹ concentrations were used). Approximately 0.2 - 0.4 ml of the solution was added to the samples on a 1 cm² of the surface, the used volume depends on the capacity of the vessel and the samples were alternatively incubated for 10 or 30 or 60 minutes.
J) The solution of secondary antibody was removed and replaced by the washing solution: 1x PBS without or with the addition alternatively 0.1 %; 0.2 %; 0.4 %; 1 % or 10 % Tween 20 (vol./vol., approximately 0.2 - 0.4 ml of solution to 1 cm² area were added, the amount used was derived from the capacity of the vessel) and the samples were incubated 10 minutes. This step was repeated three more times. In some embodiments, a solution of water without or with the addition of Tween 20 or the solution of 25 mmol.l⁻¹ Tris-HCl, pH 7.4 and 150 mmol.l⁻¹ NaCl without or with the addition of Tween 20 (the used concentrations were the same as in the usage of 1x PBS) was used instead of 1x PBS. In the further embodiments, , Triton X-100 (alternatively in the concentration of 0.1 % or 0.2 % (vol./vol.)) or SDS or LiDS or sarkosyl, all alternatively in concentrations of 0.01 %; 0.02 %; 0.03 %; 0.04 % or 0.05 % (weight/vol.) was used instead of Tween 20.
K) The washing solution was removed and replaced with a solution of proteinase K (approximately 0.2 - 0.4 ml of solution for 1 cm² of area were added, the amount used was derived from the capacity of the vessel) and the samples were incubated 30 or 60 minutes on a laboratory shaker at 300 rpm at 37 °C. Alternatively, the samples were incubated at a temperature of 56 °C.
L) 10 % or 20 % of a solution of the elution substances (SDS or LiDS or sarkosyl (weight/vol.) was alternatively added to the solution of proteinase K, the size of the added volume was alternatively equal to one-ninth, to a quarter or to the whole volume of the solution of proteinase K). The samples were incubated on a laboratory shaker at 300 rpm for a period of alternatively 10, 30 or 60 minutes.
M) 120 µl of the solution was removed and transferred to the wells of the black 96-well plate. The signal was measured using plate reader, preferably at an excitation wave length of 354 - 362 nm in the case of DAPI, at an excitation wave length of 346 - 354 nm in the case of Hoechst 33342 and Hoechst 33258 and at an excitation wave length of 388 - 396 in the case of Hoechst 34580 and also preferably at an emission wave length of 451 - 471 nm for DAPI, Hoechst 33342 and Hoechst 33258 and an emission wave length of 430 - 450 nm with Hoechst 34580. The correction of the background in the case of signal provided by DAPI and/or Hoechst was performed after measuring the signal coming from the Petri dishes, or culture flasks, or 12-, or 24-, or 48-, 96-well plates, or glasses without cells. In the case of the performance of several repetitions, the average signal in the wells of the Petri dishes or culture flasks or 12-, or 24-, or 48-, 96- well plates, or glasses without cells was preferably determined and the average values of these signals were worked with. This signal was subsequently subtracted from the individual signals of the samples with cells. The relative values of this corrected DAPI and/or Hoechst signals reflects then the relative values of the number of cells in the individual wells. In the case that it was necessary to carry out a precise determination of the cells in the individual samples, the number of cells in some samples was determined at the same time using a microscope and CCD camera and the average DAPI and/or Hoechst signal in a single cell was subsequently calculated. The corrected signals of DAPI and/or Hoechst provided by the individual samples were then divided by this signal and the number of cells in the individual samples was determined. Alternatively, the samples of serially diluted samples of cells which were processed according to the described approach, the calibration curve was calculated and subsequently the number of cells in the individual samples was determined.

The signal provided by antibody conjugated with fluorochrome Alexa Fluor 488 was also measured using plate reader at the excitation wave length of 490 - 498 nm and emission wave length of 510 - 530 nm. Subsequently, the correction of the background of this signal was or was not conducted. The determination of the mutual amount of protein and the possible correction were conducted in several ways:
- In the case that the correction of the background of the signal provided by the antibody conjugated with fluorochrome was not conducted, the signal provided by the antibody conjugated with fluorochrome was divided by the corrected signal provided by DAPI and/or Hoechst and/or the calculated number of cells. In some embodiments, in the case of the performance of several repetitions, the average signal provided by the antibody conjugated with fluorochrome and the average corrected signal provided by DAPI and/or Hoechst and/or the average number of cells in these samples were determined and only subsequently was the average signal provided by the antibody conjugated with fluorochrome divided by the average corrected signal provided by DAPI and/or Hoechst and/or the average number of cells. The calculated value was then used for a comparison of the mutual amount of protein in various samples. This method is suitable primarily in the case when the used antibodies raised against the tracked protein and/or antibodies conjugated with fluorochrome does not bind strongly on the cellular structures, and/or on the Petri dishes, or on the cultivation flasks, or on the 12-, or 24-, or 48-, 96-well plates, or on the glasses, and/or they can be easily removed by washing.
- In the case that it was necessary to carry out a correction on the background, it was proceeded in two different ways.

In the first case, the signal provided by the antibody conjugated with fluorochrome in the samples with cells, which were cultivated with both primary and secondary antibody and in the samples with cells, where the incubation with the primary antibody was omitted, was first measured. Subsequently, the signal in the samples with cells incubated with both primary and secondary antibody and in the samples with cells, where the incubation with the primary antibody was omitted was divided by the value of the corrected signal of DAPI and/or Hoechst and/or the calculated number of cells. In the case when several repetitions of the samples with cells incubated and not incubated with primary antibody was carried out, preferably, the average signal provided by the antibody conjugated with a fluorochrome and also the average corrected the signal provided by DAPI and/or Hoechst and/or the average number of cells from these repetitions was calculated first and this was then used in these and the subsequent calculations. This method was especially suitable in the case of samples with cells, which were not incubated with primary antibody. Subsequently, the signal provided by the antibody conjugated with fluorochrome was divided by the corrected signal provided by DAPI and/or Hoechst and/or the calculated number of cells. The calculated values for samples with cells, which were not incubated with primary antibody, were subtracted from the values calculated for the samples with cells incubated with primary antibody. The calculated values were, if it was necessary and did not arise directly from the previous calculation, averaged for the individual repetitions. The calculated values were used for a comparison of the mutual amount of the tracked protein detected in various samples. This method is suitable primarily in the case when the main part of the background is derived from the strong bond of the antibodies to the cellular components and only a small part of the background is caused by their bond to the Petri dishes, or cultivation flasks, or 12-, or 24-, or 48-, 96-well plates, or glasses.

In the second case, the signal provided by the antibody conjugated with fluorochrome in the samples with cells, which were not incubated with primary antibody and simultaneously in the samples with cells, which were incubated with primary antibody, was first measured. Furthermore, the signal in samples without cells was measured. The signal in samples without cells was subsequently subtracted from the signal provided by the antibody conjugated with fluorochrome in samples with cells. In the case that the samples without cells were processed in several repetitions, the average of the measured values of the signals was preferably used for the further calculations. Subsequently, the calculated values of the signals in the samples with cells not incubated with primary antibody and in the samples with cells incubated with primary antibody were divided by the value of the corrected signal of DAPI and/or Hoechst and/or the calculated number of cells. In an embodiment, when several repetitions were performed of samples with cells incubated and not incubated with primary antibody, the average signal of provided by the antibody conjugated with fluorochrome and also the average corrected signal provided by DAPI and/or Hoechst and/or the average number of cells from these repetitions was preferably calculated first and this was used in these and the subsequent calculations. This method was especially suitable in the case of the samples with cells, which were not incubated with primary antibody. Subsequently, the signal provided by the antibody conjugated with fluorochrome was divided by the corrected signal provided by DAPI and/or Hoechst and/or the calculated number of cells. The calculated values for samples with cells without the primary antibody were subtracted from the values calculated for the samples with primary antibody. The calculated values were, if it was necessary and did not arise directly from the previous calculation, averaged for the individual repetitions. The calculated values were used for a comparison of the amount of proteins in various samples. This method is suitable primarily in the case when the main part of the background is derived from strong bond of antibodies to the Petri dishes, or cultivation flask, or 12-, or 24-, or 48-, 96-well plates, or glasses.

In some embodiments, elution substances were added to the solution of proteinase K or the step with proteinase K was omitted. The concentration of the elution substances in this step was alternatively 0.1 %, 0.2 %; 1 %; 2 %; 5 %; 10 % or 20 % (weight/vol.) and the samples were incubated on a laboratory shaker at 300 rpm for a period of alternatively 10, 30 or 60 minutes.

In other embodiments, 120 µl of the sample was transferred immediately after incubation with proteinase K into the wells of black 96-well plates and only then was the solution of the elution substances added.

In some embodiments, methanol was used instead of ethanol or a fixation with formaldehyde and permeabilization with Triton X-100 was used following Example 6.

### Example 15

Optimization of the content of elution substances.

As it was previously determined, the presence of SDS leads to an increase of fluorescence both DAPI and Hoechst (Nucleic Acids Res., 5, 3775-3799; Photochem. Photobiol., 73, 339-348). It was, nevertheless, necessary to determine at which concentrations of the elution substances the signal will be the highest, whether it will be sufficient for the precise determination of the DNA, whether it will not be influenced by the presence of the DNA, or other parts of the sample, whether there is a cross-section of the concentration, which is necessary for the washing out and a concentration for the sufficient increase of the signal and how linear the final signal will be in terms of the amount of DNA in a sample. Generally, in the method it is not important the increase of the fluorescence comparing to the state without the elution substance added, but the absolute value measured. Optimally, it was necessary for the maximal fluorescent signal to overlap with values, which are necessary for the elution of DAPI and/or Hoechst from the DNA of the samples, and, at the same time, that the fluorescent signal would linearly increase with the increasing concentration of DNA. The analysis of the signal of DAPI in various concentrations of SDS showed a high absolute fluorescence already with values around 0.3125 % (weight/vol.) of SDS. In the case of sarkosyl and DAPI, the high value of the absolute fluorescence was measured at 0.625 % (weight/vol.) of sarkosyl. The signal in SDS or sarkosyl did not change further more distinctly (Figures 1 and 2). We obtained a similar result as in the case of SDS also in the case of LiDS. From this perspective, concentrations above 0.3 % (weight/vol.) of SDS or LiDS or concentrations over 0.6 % (weight/vol.) of sarkosyl are suitable for the elution of DAPI. Moreover, it was clear that the signal of DAPI in SDS is generally more than 2x higher than the signal measured in sarkosyl (Figure 3). It was similar also in the case of the ratio of the signals of DAPI in LiDS and sarkosyl. The signal of Hoechst also showed a dependence on the concentrations of the elution substances (Figures 1 and 2). The highest signal of Hoechst was measured at concentrations of 5 to 10 % (weight/vol.) of SDS. It was similar in the case of LiDS. The highest signal of Hoechst in solutions containing sarkosyl was measured at concentration of around 0.6 % (weight/vol.) of sarkosyl. With the exception of 0.625 % concentration, the signal of Hoechst in SDS was higher than its signal in sarkosyl (Figure 3). It was also similar in the case of LiDS.

### Solutions and chemicals used:

### 1. Phosphate-buffered saline (PBS)

10x PBS (a ten times concentrated solution of PBS):
1.4 mol.l⁻¹ of NaCl
26 mmol.l⁻¹ of KCl
90 mmol.l⁻¹ of Na₂HPO₄
4 mmol.l⁻¹ of KH₂PO₄

The listed components were dissolved in distilled water and the pH was adjusted to the range of 7.3-7.41x PBS (phosphate-buffered saline in the usual concentration)

### 2. Solution of monovalent copper

Two methods were used for the preparation of an aqueous solution of monovalent copper.

In the first method, the mixture was prepared by mixing the same amounts of 8 mmol.l⁻¹ of an aqueous solution of copper (II) sulphate and 20 mmol.l⁻¹ of an aqueous solution of sodium ascorbate and 40 mmol.l⁻¹ of an aqueous solution of glycine. The mixture was immediately used after mixing.

In the second method, the mixture was prepared by mixing the same amounts of 4 mmol.l⁻¹ of an aqueous solution of copper sulphate and 20 mmol.l⁻¹ of an aqueous solution of hydroquinone. The mixture was immediately used after mixing.

### 3. Solution of proteinase K

The solution of proteinase K was prepared from the stock solution of proteinase K (20 mg.ml⁻¹, ThermoFisher Scientific) diluted in 50 mmol.l⁻¹ of Tris, pH 8. The final solutions alternatively contained 0.05; 0.1; 0.2; 1 and 2 mg.ml⁻¹ of proteinase K.

### 4. Solution of monovalent copper with azide fluorochrome

The solution of monovalent copper and azide fluorochrome contained:
2 mmol.l⁻¹ of copper (II) sulphate
10 mmol.l⁻¹ of hydroquinone
10 µmol.l⁻¹ of azide fluorochrome (5-carboxyfluorescein; 5-FAM)

### Industrial Applicability

The method can be used in the fields of the study of the toxic activity of substances e.g. in the development of new drugs or in laboratories dealing with cellular cultivations, technologies utilizing DNA and the impact of various substances on cell growth. The method is suitable as a part of kits for determining the amount of DNA and cells.

## Claims

1. A method of determining the amount of DNA in a sample, **characterized in that** it contains the following steps:
(i) a sample is incubated in a solution of at least one fluorescent substance selected from the group consisted of 4',6-diamidino-2-phenylindole, 2-(4-hydroxyphenyl)-5-(4-methyl-1-piperazinyl)-2,5'-bi-1H-benzimidazole, 2'-(4-ethoxyphenyl)-5-(4-methyl-1 -piperazinyl)-2,5 '-bi-1H-benzimidazole and N,N-dimethyl-4-[5-(4-methyl-1-piperazinyl)[2,5-bi-1H-benzimidazole]-2'-yl]benzenamine, which, in the course of this incubation, binds to the DNA present in the sample;
(ii) the sample is washed with a washing solution and/or water, which washes away the fluorescent substances not bound to the DNA in the sample;
(iii) the fluorescent substances bound to the DNA in the sample are eluted from the DNA with an aqueous elution solution of at least one substance selected from the group consisted of sodium dodecylsulphate, lithium dodecylsulphate and sodium [dodecanoyl(methyl)amino] acetate, wherein the concentration of sodium dodecylsulphate, lithium dodecylsulphate and/or sodium [dodecanoyl(methyl)amino]acetate is at least 0.1 % (weight/v);
(iv) the amount of DNA is determined in the elution solution from the previous step by measuring the fluorescent signal of the fluorescent substance used in step (i).

2. The method of determining the amount of DNA in a sample according to claim 1,
**characterized in that** the concentration of the solution of the at least one fluorescent substance in step (i) is in the range of from 0.1 to 20 µmol·l-1, and it contains water and/or methanol and/or ethanol and/or dimethyl sulphoxide, preferably the solution is aqueous solution.

3. The method of determining the amount of DNA in a sample according to claim 1 or 2,
**characterized in that** the washing solution and/or water in step (ii) contains a buffer of the resulting pH 6 to 8, and/or it comprises at least one detergent, selected from the group comprising polyoxyethylenesorbitan monolaurate, polyoxyethylenesorbitan monooleate, 4-(1,1,3,3-tetramethylbutyl)phenyl-polyethylene glycol, 4-Nonylphenyl-polyethylene glycol, sodium dodecylsulphate, lithium dodecylsulphate, sodium [dodecanoyl(methyl)amino]acetate.

4. The method of determining the amount of DNA in a sample according to claim 3,
**characterized in that** the washing solution and/or water comprises of at least one detergent, and the concentration of the detergent in the washing solution and/or water is in the range of from 0.01 % (vol./vol.) to 10 % (vol./vol.), and in the case of sodium dodecylsulphate, lithium dodecylsulphate and sodium [dodecanoyl(methyl)amino] acetate, their concentration in the washing solution is in the range of from 0.01 % (weight/vol.) to 0.05 % (weight/vol.).

5. The method of determining the amount of DNA in a sample according to any one of the preceding claims, **characterized in that** before the step (i) of incubation with the fluorescent substance and/or before the step (ii) of washing the sample with the washing solution and/or water, and/or before the step (iii) of elution of the sample using elution solution, incubation of the sample with RNase, preferably with RNase A is performed.

6. The method of determining the amount of DNA in a sample according to any one of the preceding claims, **characterized in that** the sample is cells or tissues or anchored DNA, wherein in the case that the sample is cells or tissues, the sample is preferably fixed.

7. The method of determining the amount of DNA in a sample according to any one of the preceding claims, **characterized in that** the incubation of the sample with at least one fluorescent substance in step (i) is conducted at a temperature of 0 to 45 °C for a period longer than 2 minutes; the washing of the sample with the washing solution and/or water in step (ii) is performed preferably at a temperature of 0 to 45 °C for a period longer than 10 minutes, wherein in the course of this time the washing solution and/or water is changed at least 2x for a new one, more preferably this step is conducted for a period of 15 to 60 minutes and the washing solution and/or water is changed for a new one at least 3x; the elution of the fluorescent substances from the DNA in step (iii) is performed preferably at a temperature of 0 to 45 °C for a period longer than 2 minutes.

8. The method of determining the amount of DNA in a sample according to any one of the preceding claims, **characterized in that** if the samples are cells or a tissue, then before the step (i) or during the step (i) or before the step (ii) or before the step (iii), incubation of the sample in an aqueous solution containing monovalent copper ions is performed.

9. The method of determining the amount of DNA in a sample according to any one of the preceding claims, **characterized in that** if the sample is cells or a tissue, then between the steps (ii) and (iii) or simultaneously with the step (iii) the sample is incubated with proteinase K.

10. A method of determining the amount of 5-ethynyl-2'-deoxyuridine and/or 5-bromo-2'-deoxyuridine and/or 5-chloro-2'-deoxyuridine and/or 5-iodo-2'-deoxyuridine incorporated into DNA and/or the content of proteins and/or the amount of RNA and/or the amount of specific DNA sequences and/or the amount of sugars and/or the amount of lipids and/or the amount of fluorescently-labelled DNA and/or fluorescently-labelled RNA and/or fluorescently-labelled proteins in the cells and/or tissues using the method according to any one of the preceding claims, **characterized in that** the incubation of cells and/or tissues with a fluorescent probe is performed, in the course of which the fluorescent probe binds to the 5-ethynyl-2'-deoxyuridine and/or 5-bromo-2'-deoxyuridine and/or 5-chloro-2'-deoxyuridine and/or 5-iodo-2'-deoxyuridine incorporated into DNA and/or to the protein and/or to RNA and/or to specific DNA sequence and/or to the sugar and/or to the lipid and/or to the fluorescently-labelled DNA and/or fluorescently-labelled RNA and/or fluorescently-labelled proteins; wherein the fluorescent probe are compounds selected from the group of fluorochromes and fluorochromes conjugated with antibodies, lectins, RNA, DNA or azide functional groups,
wherein before, in the course of or after the reaction with the fluorescent probe, the steps (i) and (ii) of the method according to any one of the preceding claims are performed,
and, subsequently, after the reaction with the fluorescent probe as well as after performing the steps (i) and (ii) of the method according to any one of the preceding claims, the step (iii) of the method according to any one of the preceding claims is performed;
subsequently, the fluorescence signal provided by the fluorescent probes and/or by the fluorescently-labelled DNA and/or fluorescently-labelled RNA and/or fluorescently-labelled proteins is measured, as well as the fluorescence signal provided by the fluorescent substance selected from the group consisted of 4',6-diamidino-2-phenylindole, 2'-(4-hydroxyphenyl)-5-(4-methyl-1-piperazinyl)-2,5'-bi-1H-benzimidazole, 2'-(4-ethoxyphenyl)-5-(4-methyl-1-piperazinyl)-2,5'-bi-1H-benzimidazole and N,N-dimethyl-4-[5-(4-methyl-1-piperazinyl)[2,5'-bi-1H-benzimidazole]-2'-yl]benzenamine is measured;
thereafter, the signal provided by the fluorescent probes and/or by the fluorescently-labelled DNA and/or fluorescently-labelled RNA and/or fluorescently-labelled proteins is divided by the signal provided by the fluorescent substance, corresponding to the number of cells in the sample, and the amount of 5-ethynyl-2'-deoxyuridine and/or 5-bromo-2'-deoxyuridine and/or 5-chloro-2'-deoxyuridine and/or 5-iodo-2'-deoxyuridine incorporated into DNA and/or the content of proteins and/or the amount of RNA and/or the amount of specific DNA sequences and/or the amount of sugars and/or the amount of lipids and/or the amount of fluorescently-labelled DNA and/or fluorescently-labelled RNA and/or fluorescently-labelled proteins in cells or tissues of the sample is determined.

11. The method according to claim 10, **characterized in that** before the incubation step with a fluorescent probe, the sample of cells or tissues undergoes a reaction with an antibody, which subsequently reacts with the fluorescent probe.

12. A method of determining the replication activity in cells and tissues using the method according to any one of the claims 1 to 9, **characterized in that** the sample of cells or tissues is first incubated with 5-ethynyl-2'-deoxyuridine and/or 5-bromo-2'-deoxyuridine and/or 5-chloro-2'-deoxyuridine and/or 5-iodo-2'-deoxyuridine;
subsequently, the step (i) of incubation with the fluorescent substance selected from the group consisted of 2-(4-hydroxyphenyl)-5-(4-methyl-1-piperazinyl)-2,5-bi-1H-benzimidazole, 2'-(4-ethoxyphenyl)-5-(4-methyl-1-piperazinyl)-2,5'-bi-1H-benzimidazole and N,N-dimethyl-4-[5-(4-methyl-1-piperazinyl)[2,5'-bi-1H-benzimidazole]-2'-yl]benzenamine, and the step (ii) of washing with washing solution and/or water of the method according to any one of the claims 1 to 9 are performed; subsequently, an incubation of the sample with proteinase K is conducted; then the fluorescent signal provided by the fluorescent substance from step (i) is measured; subsequently, step (iii) of the method according to any one of the claims 1 to 9 is performed, and the signal provided by the fluorescent substance from step (i) is measured again; subsequently, the fluorescence signal provided by the fluorescent substance after incubation with proteinase K is compared with the fluorescence signal provided by the fluorescent substance contained in the elution solution.

## Patentansprüche

1. Ein Verfahren zur Bestimmung der DNA-Menge in einer Probe, **dadurch gekennzeichnet, dass** es die folgenden Schritte enthält:
(i) Eine Probe wird inkubiert in einer Lösung von mindestens einer fluoreszierenden Substanz, ausgewählt aus der Gruppe bestehend aus 4',6-Diamidino-2-phenylindol, 2'-(4-Hydroxyphenyl)-5-(4-methyl-1-piperazinyl)-2,5'-bi-1H-benzimidazol, 2'-(4-Ethoxyphenyl)-5-(4-methyl-1-piperazinyl)-2,5'-bi-1H-benzimidazol und N,N-Dimethyl-4-[5-(4-methyl-1-piperazinyl)[2,5'-bi-1H-benzimidazol]-2'-yl]benzolamin, das im Verlauf dieser Inkubation an die in der Probe vorgehandene DNA bindet;
(ii) die Probe wird mit einer Waschlösung und/oder Wasser gewaschen, die die fluoreszierenden Substanzen, die nicht an die DNA in der Probe gebunden sind, wegwaschen;
(iii) die fluoreszierenden Substanzen, die an die DNA in der Probe gebunden sind, mit einer wässrigen Elutionslösung von mindestens einer Substanz ausgewählt aus der Gruppe bestehend aus Natriumdodecylsulfat, Lithiumdodecylsulfat und Natrium[dodecanoyl(methyl)amino]acetat aus der DNA eluiert werden, wobei die Konzentration von Natriumdodecylsulfat, Lithiumdodecylsulfat und/oder Natrium[dodecanoyl(methyl)amino]acetat mindestens 0,1% (Gew./Vol.) beträgt;
(iv) die Menge an DNA wird in der Elutionslösung aus dem vorherigen Schritt durch Messen des Fluoreszenzsignals der in Schritt (i) verwendeten fluoreszierenden Substanz bestimmt.

2. Verfahren zur Bestimmung der DNA-Menge in einer Probe nach Anspruch 1, **dadurch gekennzeichnet, dass** die Konzentration der Lösung der mindestens einen fluoreszierenden Substanz in Schritt (i) im Bereich von 0,1 bis 20 µmol·l⁻¹ liegt und die Lösung enthält Wasser und/oder Methanol und/oder Ethanol und/oder Dimethylsulfoxid, vorzugsweise ist die Lösung eine wässrige Lösung.

3. Verfahren zur Bestimmung der DNA-Menge in einer Probe nach Anspruch 1 oder 2, **dadurch gekennzeichnet, dass** die Waschlösung und/oder das Wasser in Schritt (ii) einen Puffer mit einem resultierenden pH-Wert von 6 bis 8 enthält und/oder mindestens einen Waschmittel, ausgewählt aus der Gruppe bestehend aus Polyoxyethylensorbitanmonolaurat, Polyoxyethylensorbitanmonooleat, 4-(1,1,3,3-Tetramethylbutyl)phenylpolyethylenglykol, 4-Nonylphenylpolyethylenglykol, Natriumdodecylsulfat, Lithiumdodecylsulfat und Natrium[dodecanoyl(methyl)amino]acetat enthält.

4. Verfahren zur Bestimmung der DNA-Menge in einer Probe nach Anspruch 3, **dadurch gekennzeichnet, dass** die Waschlösung und/oder das Wasser mindestens ein Waschmittel enthält und die Konzentration des Waschmittels in der Waschlösung und/oder dem Wasser im Bereich von 0,01% (Vol./Vol.) bis 10% (Vol./Vol.) liegt, und im Falle von Natriumdodecylsulfat, Lithiumdodecylsulfat und Natrium[dodecanoyl(methyl)amino]acetat liegt die Konzentration in der Waschlösung im Bereich von 0,01% (Gew./Vol.) bis 0,05% (Gew./Vol.).

5. Verfahren zur Bestimmung der DNA-Menge in einer Probe nach einem der vorhergehenden Ansprüche, **dadurch gekennzeichnet, dass** vor dem Schritt (i) der Inkubation mit der fluoreszierenden Substanz, und/oder vor dem Schritt (ii) des Waschens der Probe mit der Waschlösung und/oder Wasser, und/oder vor dem Schritt (iii) der Elution der Probe unter Verwendung der Elutionslösung, wird eine Inkubation der Probe mit RNase, vorzugsweise mit RNase A, durchgeführt.

6. Verfahren zur Bestimmung der DNA-Menge in einer Probe nach einem der vorhergehenden Ansprüche, **dadurch gekennzeichnet, dass** die Probe Zellen oder Gewebe oder verankerte DNA ist, wobei in dem Fall, dass die Probe Zellen oder Gewebe ist, die Probe vorzugsweise fixiert ist.

7. Verfahren zur Bestimmung der DNA-Menge in einer Probe nach einem der vorhergehenden Ansprüche, **dadurch gekennzeichnet, dass** die Inkubation der Probe mit mindestens einer fluoreszierenden Substanz in Schritt (i) bei einer Temperatur von 0 bis 45 °C durchgeführt wird über einen Zeitraum von mehr als 2 Minuten; das Waschen der Probe mit der Waschlösung und/oder Wasser in Schritt (ii) wird vorzugsweise bei einer Temperatur von 0 bis 45 °C über einen Zeitraum von mehr als 10 Minuten durchgeführt, wobei im Laufe dieser Zeit die Waschlösung und/oder oder Wasser wird mindestens zweimal gegen neues ausgetauscht, bevorzugter wird dieser Schritt für einen Zeitraum von 15 bis 60 Minuten durchgeführt und die Waschlösung und/oder Wasser wird mindestens dreimal gegen neues ausgetauscht; die Elution der fluoreszierenden Substanzen aus der DNA in Schritt (iii) wird vorzugsweise bei einer Temperatur von 0 bis 45 °C über einen Zeitraum von mehr als 2 Minuten durchgeführt.

8. Verfahren zur Bestimmung der DNA-Menge in einer Probe nach einem der vorhergehenden Ansprüche, **dadurch gekennzeichnet, dass,** wenn die Probe Zellen oder ein Gewebe ist, denn vor dem Schritt (i) oder während des Schritts (i) oder vor dem Schritt (ii) oder vor dem Schritt (iii) wird eine Inkubation der Probe in einer wässrigen Lösung, die einwertige Kupferionen enthält, durchgeführt.

9. Verfahren zur Bestimmung der DNA-Menge in einer Probe nach einem der vorhergehenden Ansprüche, **dadurch gekennzeichnet, dass,** wenn die Probe Zellen oder ein Gewebe ist, zwischen den Schritten (ii) und (iii) oder gleichzeitig mit dem Schritt (iii) die Probe wird mit Proteinase K inkubiert.

10. Verfahren zur Bestimmung der Menge an 5-Ethinyl-2'-desoxyuridin und/oder 5-Brom-2'-desoxyuridin und/oder 5-Chlor-2'-desoxyuridin und/oder 5-Iod-2'-desoxyuridin eingebaut in DNA und/oder der Gehalt an Proteinen und/oder die Menge an RNA und/oder die Menge an spezifischen DNA-Sequenzen und/oder die Menge an Zuckern und/oder die Menge an Lipiden und/oder die Menge an fluoreszenzmarkierter DNA und/oder die Menge an fluoreszierend markierte RNA und/oder die Menge an fluoreszierend markierte Proteine in den Zellen und/oder Geweben unter Verwendung des Verfahrens nach einem der vorhergehenden Ansprüche, **dadurch gekennzeichnet, dass** die Inkubation von Zellen und/oder Geweben mit einer fluoreszierenden Sonde durchgeführt wird, im Verlauf von der Inkubation die fluoreszierende Sonde gebunden ist an das 5-Ethinyl-2'-desoxyuridin und/oder 5-Brom-2'-desoxyuridin und/oder 5-Chlor-2'-desoxyuridin und/oder 5-Iod-2'-desoxyuridin in DNA und/oder an das Protein und/oder an RNA und/oder an die spezifische DNA-Sequenz und/oder an den Zucker und/oder an das Lipid und/oder an die fluoreszenzmarkierte DNA und/oder fluoreszenzmarkierte RNA und/oder fluoreszenzmarkierte Proteine; wobei die fluoreszierenden Sonden Verbindungen sind, die ausgewählt sind aus der Gruppe der Fluorochrome und Fluorochrome konjugiert mit Antikörpern, Lectinen, RNA, DNA oder Azid-funktionellen Gruppen,
wobei vor, während oder nach der Reaktion mit der fluoreszierenden Sonde die Schritte (i) und (ii) des Verfahrens nach einem der vorhergehenden Ansprüche durchgeführt werden, und anschließend nach der Reaktion mit der fluoreszierenden Sonde sowie nach der Durchführung der Schritte (i) und (ii) des Verfahrens nach einem der vorhergehenden Ansprüche, der Schritt (iii) des Verfahrens nach einem der vorhergehenden Ansprüche durchgeführt wird;
anschließend wird das von den Fluoreszenzsonden und/oder der fluoreszenzmarkierten DNA und/oder fluoreszenzmarkierten RNA und/oder fluoreszenzmarkierten Proteinen bereitgestellte Fluoreszenzsignal, sowie das von der fluoreszierenden Substanz, ausgewählt aus der Gruppe bestehend aus 4',6-Diamidino-2-phenylindol, 2'-(4-hydroxyphenyl)-5-(4-methyl-1-piperazinyl)-2,5'-bi-1H-benzimidazol, 2'-(4-Ethoxyphenyl)-5-(4-methyl-1-piperazinyl)-2,5'-bi-1H-benzimidazol und N,N-Dimethyl-4-[5-(4-methyl-1-piperazinyl)[2,5'-bi-1H-benzimidazol]-2'-yl]benzolamin, bereitgestellte Fluoreszenzsignal gemessen;
danach wird das von den fluoreszierenden Sonden und/oder von der fluoreszierend markierten DNA und/oder fluoreszierend markierten RNA und/oder fluoreszierend markierten Proteinen gelieferte Signal geteilt durch das von der fluoreszierenden Substanz gelieferte Signal, entsprechend der Anzahl der Zellen in die Probe, und die Menge an 5-Ethinyl-2'-desoxyuridin und/oder 5-Brom-2'-desoxyuridin und/oder 5-Chlor-2'-desoxyuridin und/oder 5-Iod-2'-desoxyuridin eingebaut in DNA und/oder der Gehalt an Proteinen und/oder die Menge an RNA und/oder die Menge an spezifischen DNA-Sequenzen und/oder die Menge an Zuckern und/oder die Menge an Lipiden und/oder die Menge an fluoreszenzmarkierter DNA und/oder die Menge an fluoreszenzmarkierter RNA und/oder die Menge an fluoreszenzmarkierten Proteine in Zellen oder Geweben der Probe bestimmt wird.

11. Verfahren nach Anspruch 10, **dadurch gekennzeichnet, dass** die Probe von Zellen oder Geweben vor dem Inkubationsschritt mit einer fluoreszierenden Sonde eine Reaktion mit einem Antikörper eingeht, der anschließend mit der fluoreszierenden Sonde reagiert.

12. Verfahren zur Bestimmung der Replikationsaktivität in Zellen und Geweben unter Verwendung des Verfahrens nach einem der Ansprüche 1 bis 9, **dadurch gekennzeichnet, dass** die Probe von Zellen oder Geweben zuerst mit 5-Ethinyl-2'-desoxyuridin und/oder 5-Brom-2'-Desoxyuridin und/oder 5-Chlor-2'-desoxyuridin und/oder 5-Iod-2'-desoxyuridin inkubiert wird;
anschließend der Schritt (i) der Inkubation mit der fluoreszierenden Substanz, ausgewählt aus der Gruppe bestehend aus 2'-(4-Hydroxyphenyl)-5-(4-methyl-1-piperazinyl)-2,5'-bi-1H-benzimidazol, 2'-(4-Ethoxyphenyl)-5-(4-methyl-1-piperazinyl)-2,5'-bi-1H-benzimidazol und N,N-Dimethyl-4-[5-(4-methyl-1-Piperazinyl)[2,5'-bi-1H-benzimidazol]-2'-yl]benzolamin, und der Schritt (ii) des Waschens mit Waschlösung und/oder Wasser des Verfahrens nach einem der Ansprüche 1 bis 9 durchgeführt werden; anschließend wird eine Inkubation der Probe mit Proteinase K durchgeführt; dann wird das von der fluoreszierenden Substanz aus Schritt (i) bereitgestellte Fluoreszenzsignal gemessen; anschließend wird der Schritt (iii) des Verfahrens nach einem der Ansprüche 1 bis 9 durchgeführt und das von der fluoreszierenden Substanz aus Schritt (i) gelieferte Signal erneut gemessen; anschließend wird das von der fluoreszierenden Substanz nach Inkubation mit Proteinase K bereitgestellte Fluoreszenzsignal mit dem von der in der Elutionslösung enthaltenen fluoreszierenden Substanz bereitgestellten Fluoreszenzsignal verglichen.

## Revendications

1. Un procédé de détermination de la quantité d'ADN dans un échantillon, **caractérisé en ce qu'**il contient les étapes suivantes:
(i) un échantillon est incubé dans une solution d'au moins une substance fluorescente choisie dans le groupe constitué par 4',6-diamidino-2-phénylindole, 2'-(4-hydroxyphényl)-5-(4-méthyl-1 -pipérazinyl)-2,5 '-bi-1H-benzimidazole, 2'-(4-éthoxyphényl)-5-(4-méthyl-1-pipérazinyl)-2,5'-bi-1H-benzimidazole et N,N-diméthyl-4-[5-(4-méthyl-1-pipérazinyl)[2,5'-bi-1H-benzimidazole]-2'-yl]benzénamine, qui, au cours de cette incubation, se lie à l'ADN présent dans l'échantillon;
(ii) l'échantillon est lavé avec une solution de lavage et/ou avec de l'eau, ce qui élimine les substances fluorescentes non liées à l'ADN dans l'échantillon;
(iii) les substances fluorescentes liées à l'ADN dans l'échantillon sont éluées de l'ADN avec une solution aqueuse d'élution d'au moins une substance choisie dans le groupe constitué du dodécylsulfate de sodium, dodécylsulfate de lithium et [dodécanoyle (méthyl)amino]acétate de sodium, dans lequel la concentration de dodécylsulfate de sodium, de dodécylsulfate de lithium et/ou de [dodécanoyl (méthyl) amino]acétate de sodium est d'au moins 0,1 % (poids/vol.);
(iv) la quantité d'ADN est déterminée dans la solution d'élution de l'étape précédente en mesurant le signal fluorescent de la substance fluorescente utilisée à l'étape (i).

2. Le procédé de détermination de la quantité d'ADN dans un échantillon selon la revendication 1, **caractérisé en ce que** la concentration de la solution d'au moins une substance fluorescente à l'étape (i) est entre 0,1 et 20 µmol·l⁻¹, et elle contient de l'eau et/ou du méthanol et/ou de l'éthanol et/ou du diméthylsulfoxyde, de préférence, la solution est une solution aqueuse.

3. Le procédé de détermination de la quantité d'ADN dans un échantillon selon la revendication 1 ou 2, **caractérisé en ce que** la solution de lavage et/ou l'eau à l'étape (ii) contient un tampon du pH résultant de 6 à 8, et/ou comprend au moins un détergent, choisi dans le groupe comprenant monolaurate de polyoxyéthylènesorbitan, monooléate de polyoxyéthylènesorbitan, 4-(1,1,3,3-tétraméthylbutyl)phényl-polyéthylène glycol, 4-nonylphényl-polyéthylène glycol, dodécylsulphate de sodium, dodécylsulfonate de lithium, dodécylsulfone, [dodécylsulfonate de sodium amino] acétate.

4. Le procédé de détermination de la quantité d'ADN dans un échantillon selon la revendication 3, **caractérisé en ce que** la solution de lavage et/ou l'eau comprend au moins un détergent, et que la concentration du détergent dans la solution de lavage et/ou l'eau est entre 0,01 % (vol/vol) et 10 % (vol/vol), et dans le cas du dodécylsulfate de sodium, du dodécylsulfate de lithium et du [dodécanoyl (méthyl) amino] acétate de sodium, leur concentration dans la solution de lavage est entre 0,01 % (poids/vol.) et 0,05 % (poids/vol.).

5. Le procédé de détermination de la quantité d'ADN dans un échantillon selon l'une quelconque des revendications précédentes, **caractérisé en ce qu** 'avant l'étape (i) d'incubation avec la substance fluorescente et/ou avant l'étape (ii) de lavage de l'échantillon avec la solution de lavage et/ou de l'eau et/ou avant l'étape (iii) d'élution de l'échantillon en utilisant une solution d'élution, une incubation de l'échantillon avec de la RNase, de préférence avec de la RNase A, est effectuée.

6. Le procédé de détermination de la quantité d'ADN dans un échantillon selon l'une quelconque des revendications précédentes, **caractérisé en ce que** l'échantillon est des cellules ou des tissus ou d'ADN ancré, dans lequel dans le cas où l'échantillon est des cellules ou des tissus, l'échantillon est de préférence fixé.

7. Le procédé de détermination de la quantité d'ADN dans un échantillon selon l'une quelconque des revendications précédentes, **caractérisé en ce que** l'incubation de l'échantillon avec au moins une substance fluorescente à l'étape (i) est effectuée à une température de 0 à 45 °C pendant une période supérieure à 2 minutes; le lavage de l'échantillon avec la solution de lavage et/ou l'eau à l'étape (ii) est effectué de préférence à une température de 0 à 45 °C pendant une durée supérieure à 10 minutes, la solution de lavage et/ou ou l'eau est changée au moins deux fois pour une nouvelle, plus préférablement cette étape est effectuée pendant une période de 15 à 60 minutes et la solution de lavage et/ou l'eau est changée pour une nouvelle au moins trois fois; l'élution des substances fluorescentes de l'ADN à l'étape (iii) est effectuée de préférence à une température de 0 à 45 °C pendant une période supérieure à 2 minutes.

8. Le procédé de détermination de la quantité d'ADN dans un échantillon selon l'une quelconque des revendications précédentes, **caractérisé en ce que** si les échantillons sont des cellules ou un tissu, alors avant l'étape (i) ou pendant l'étape (i) ou avant l'étape (ii) ou avant l'étape (iii), l'incubation de l'échantillon dans une solution aqueuse contenant des ions cuivre monovalents est réalisée.

9. Le procédé de détermination de la quantité d'ADN dans un échantillon selon l'une quelconque des revendications précédentes, **caractérisé en ce que** si l'échantillon est une cellule ou un tissu, alors entre les étapes (ii) et (iii) ou simultanément à l'étape (iii), l'échantillon est incubé avec la protéinase K.

10. Un procédé de détermination de la quantité de 5-éthynyl-2'-désoxyuridine et/ou de 5-bromo-2'-désoxyuridine et/ou de 5-chloro-2'-désoxyuridine et/ou de 5-iodo-2'-désoxyuridine incorporée dans ADN et/ou la teneur en protéines et/ou la quantité d'ARN et/ou la quantité de séquences spécifiques d'ADN et/ou la quantité de sucres et/ou la quantité de lipides et/ou la quantité d'ADN marqué par fluorescence et/ou protéines marquées par fluorescence ou ARN marquées par fluorescence dans les cellules et/ou tissus en utilisant le procédé selon l'une quelconque des revendications précédentes, **caractérisées en ce que** l'incubation de cellules et/ou de tissus avec une sonde fluorescente est effectuée, au cours dont la sonde fluorescente se lie à la 5-éthynyl-2'-désoxyuridine et/ou à la 5-bromo-2'-désoxyuridine et/ou à la 5-chloro-2'-désoxyuridine et/ou à la 5-iodo-2'-désoxyuridine incorporées dans l'ADN et/ou à la protéine et/ou à l'ARN et/ou à la séquence d'ADN spécifique et/ou au sucre et/ou au lipide et/ou à l'ADN marqué par le fluorescent et/ou à l'ARN marqué par le fluorescent et/ou aux protéines marquées par fluorescence; dans lesquels la sonde fluorescente est des composés choisis dans le groupe des fluorochromes et des fluorochromes conjugués à des anticorps, des lectines, des groups de fonction de l'ARN, de l'ADN ou de l'azide,
dans lequel les étapes (i) et (ii) du procédé selon l'une quelconque des revendications précédentes sont effectuées avant, au cours de ou après la réaction avec la sonde fluorescente, et, après la réaction avec la sonde fluorescente et après avoir effectué les étapes (i) et (ii) du procédé selon l'une quelconque des revendications précédentes, l'étape (iii) du procédé selon l'une quelconque des revendications précédentes est ensuite effectuées;
ensuite, on mesure le signal de fluorescence fourni par les sondes fluorescentes et/ou par l'ADN marqué par fluorescence et/ou l'ARN marqué par fluorescence et/ou les protéines marquées par fluorescence, ainsi que le signal de fluorescence fourni par la substance fluorescente choisie parmi composé de 4',6-diamidino-2-phénylindole, 2'-(4-hydroxyphényl)-5-(4-méthyl-1-pipérazinyl)-2,5'-bi-1H-benzimidazole, 2'-(4-éthoxyphényl)-5-(4-méthyl-1-pipérazinyl)-2,5'-bi-1H-benzimidazole et le N,N-diméthyl-4-[5-(4-méthyl-1-pipérazinyl) [2,5'-bi-1H-benzimidazole]-2'-yl]benzèneamine;
ensuite, le signal fourni par les sondes fluorescentes et/ou par l'ADN marqué par fluorescence et/ou par l'ARN marqué par fluorescence et/ou par protéines marquées par fluorescence est divisé par le signal fourni par la substance fluorescente, correspondant au nombre de cellules dans l'échantillon, et la quantité de 5-éthynyl-2'-désoxyuridine et/ou de 5-bromo-2'-désoxyuridine et/ou de 5-chloro-2'-désoxyuridine et/ou de 5-iodo-2'-désoxyuridine incorporée dans ADN et/ou la teneur en protéines et/ou la quantité d'ARN et/ou la quantité de séquences spécifiques d'ADN et/ou la quantité de sucres et/ou la quantité de lipides et/ou la quantité d'ADN marqué par fluorescence et/ou la quantité d'ARN marqué par fluorescence et/ou des protéines marquées par fluorescence dans des cellules ou des tissus de l'échantillon sont déterminées.

11. Le procédé selon la revendication 10, **caractérisé en ce que** avant l'étape d'incubation avec une sonde fluorescente, l'échantillon de cellules ou de tissus subit une réaction avec un anticorps, qui réagit ensuite avec la sonde fluorescente.

12. Un procédé de détermination de l'activité de réplication dans des cellules et des tissus en utilisant le procédé selon l'une quelconque des revendications 1 à 9, **caractérisé en ce que** l'échantillon de cellules ou de tissus est d'abord incubé avec 5-éthynyl-2'-désoxyuridine et/ou 5-bromo-2'-désoxyuridine et/ou 5-chloro-2'-désoxyuridine et/ou 5-iodo-2'-désoxyuridine; ensuite, l'étape (i) d'incubation avec la substance fluorescente choisie dans le groupe consistant de 2'-(4-hydroxyphényl)-5-(4-méthyl-1-pipérazinyl)-2,5'-bi-1H-benzimidazole, 2'-(4-éthoxyphényl)-5-(4-méthyl-1-pipérazinyl)-2,5'-bi-1H-benzimidazole et N, N-diméthyl-4-[5-(4-méthyl-1-pipérazinyl)[2,5'-bi-1H-benzimidazole]-2'-yl]benzèneamine, et l'étape (ii) de lavage avec une solution de lavage et/ou de l'eau du procédé selon l'une quelconque des revendications 1 à 9 sont effectuées; ensuite, une incubation de l'échantillon avec la protéinase K est réalisée; ensuite, le signal fluorescent fourni par la substance fluorescente de l'étape (i) est mesuré; ensuite, l'étape (iii) du procédé selon l'une quelconque des revendications 1 à 9 est réalisée et le signal fourni par la substance fluorescente issue de l'étape (i) est à nouveau mesuré; ensuite, le signal de fluorescence fourni par la substance fluorescente après incubation avec la protéinase K est comparé au signal de fluorescence fourni par la substance fluorescente contenue dans la solution d'élution.
